(19) Europäisches Patentamt
European Patent Office
Office européen des brevets

(11) EP 1 477 475 A1

(12) **EUROPEAN PATENT APPLICATION**

(43) Date of publication:
**17.11.2004 Bulletin 2004/47**

(21) Application number: **03011263.5**

(22) Date of filing: **16.05.2003**

(51) Int Cl.[7]: **C07C 233/81**, C07D 403/12, C07D 407/12, A61K 31/167, A61K 31/341, A61K 31/4035, G06F 17/00, G06F 19/00

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(71) Applicant: **Procorde GmbH**
**82152 Martinsried (DE)**

(72) Inventors:
- **Rosport Kai**
  **81731 München (DE)**
- **Ungerer Martin**
  **80799 München (DE)**
- **Münch Götz**
  **80799 München (DE)**
- **Quitterer Ursula**
  **97078 Würzburg (DE)**
- **Lohse Martin**
  **97074 Würzburg (DE)**
- **Kramer Bernd**
  **52080 Aachen (DE)**
- **Dormeyer Matthias**
  **80637 München (DE)**

(74) Representative: **Hartz, Nikolai F., Dr. et al**
**Wächtershäuser & Hartz**
**Patentanwälte**
**Weinstrasse 8**
**80333 München (DE)**

(54) **Compounds for use as a medicine increasing the contractility of a heart, a heart muscle or cells of a heart muscle**

(57) The present invention relates to a class of compounds useful in the treatment of congestive heart failure by increasing the contractility of a heart, a heart muscle or cells of a heart muscle. Moreover, the present invention relates to methods of producing a computer readable database containing representations of specific compounds of the present invention which interact with a specific binding pocket of Gβγ. The compounds or a pharmaceutically acceptable salt or ester thereof for use as a medicine of the invention are characterised by the following formula (I):

A-L-B (I)

wherein A and B, which may be the same or different are represented by the following group (II)

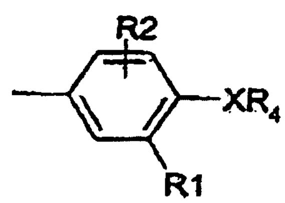

(II)

wherein X represents -CONR3-, -NR3CO-, or -NR3COCH=CH-; L represents a single bond, an oxygen atom, a sulfur atom, a $CR^5R^6$ group or a $NR^5$ group wherein $R^5$ and $R^6$ are selected from a hydrogen atom, an alkyl group and a halogen atom and R1 and R2 are defined as herein.

EP 1 477 475 A1

## Description

### Technical Field of the invention

The present invention relates to a class of compounds useful in the treatment of congestive heart failure. In particular, the present invention relates to compounds useful for increasing the contractility of a heart, a heart muscle or cells of a heart muscle. Moreover, the present invention relates to methods of producing a computer readable database containing representations of specific compounds of the present invention which interact with a specific binding pocket of Gβγ.

### Background of the invention

Congestive heart failure is a chronic disease affecting about 5 million people in the United States. The five year-mortality rate of patients suffering from congestive heart failure is presently at a level of 50 % whereby specific forms or additional complications lead to drastically increased mortality rates. Congestive heart failure develops when the heart must cope for an extended period of time with an abnormally high demand upon cardiac contractility. An abnormally high demand may be caused by cardiovascular disease such as hypertension and myocardial ischemia, cardiomyopathy or congenital heart disease.

Drugs for the treatment of congestive heart failure, which show a positive inotropic effect are known. However, conventional positive inotropic drugs do not lead to a decrease of the mortality rates. To the contrary, studies have shown that the administration of conventional drugs may even result in an increase of the mortality rates as shown by the PROMISE trial, Packer *etal*., N. Engl. J. Med. 1991; 325:1468-1475. Conventional positive inotropic drugs stimulate cAMP levels in cells.

Therefore, the development of alternative therapies for the treatment of congestive heart failure and the identification of agents having positive inotropic effects while not increasing mortality rates is highly desirable.

Gβγ is a dimeric protein complex of G proteins which act as signal transducers of many membrane-bound receptors. G proteins are membrane-bound heterotrimeric protein complexes consisting of a GTP/GDP-binding α subunit and the tightly bound Gβγ complex. G protein mediated signaling is subject to a variety of regulatory controls. Although control is mostly exerted at the receptor level, several proteins have been described to alter the activity of G proteins by direct interaction.

Phosducin is an example for a Gβγ binding protein which regulates G protein signalling (Bauer et al., 1992; Lee et al., 1992). Phosducin is present in the retina and the pineal gland (Reig 1990). Moreover, phosducin has also been purified from brain (Bauer et al., 1992), and mRNA and protein expression have been detected in other tissues (Bauer et al., 1992; Danner and Lohse, 1996). Phosducin binding to Gβγ is known from Gaudet et al. 1996 and WO 98/040402.

WO03/022882 discloses a method of increasing the contractility of a heart, a heart muscle or cells of a heart by administering an active phosducin protein for interaction with Gβγ. Since proteins are often associated with problems of stability *in vivo,* non-biological small molecules would be preferred as active compounds.

### Summary of the invention

It is a problem of the invention to provide compounds for use as a medicine in the treatment of congestive heart failure, which increase the contractility of a heart, a heart muscle or cells of a heart muscle preferably without stimulating cAMP levels of the cells.

It is a further problem of the present invention to provide pharmaceutical compositions comprising a compound of the invention.

Moreover, it is a further problem of the present invention to provide a computer readable database comprising compounds of the present invention, preferably interacting with a specific binding pocket of Gβγ.

The present invention provides a compound of of the following formula (I) or a pharmaceutically acceptable salt thereof for use as a medicine:

$$A\text{-}L\text{-}B \qquad (I)$$

wherein A and B, which may be the same or different are represented by the following group (II)

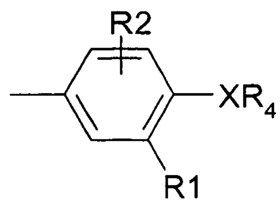

(II)

wherein

X represents -CONR3-, -NR3CO- or -NR3COCH=CH-;

L represents a single bond, an oxygen atom, a sulfur atom, a $CR^5R^6$ group or a $NR^5$ group wherein $R^5$ and $R^6$ are selected from a hydrogen atom, an alkyl group and a halogen atom, R1 and R2 which may be the same or different represent a hydrogen atom, a carboxyl group, a carboxamide group, a group COOR7, wherein R7 represents a $C_{1-4}$ alkyl group, an aryl group, an aralkyl group,

R3 represents a hydrogen atom, a $C_{1-4}$ alkyl group which may be substituted by a halogen atom or an acyl group; R4 represents an aromatic carbocyclic or heterocyclic ring, which may be substituted by one or more substituents selected from the group of halogen atoms, $C_{1-4}$ alkyl groups, a hydroxyl group, alkoxy groups, amino group, aminoalkyl group, dialkylamino group, carboxyl group, alkoxy carbonyl groups, alkylamino carbonyl groups, dialkylamino carbonyl groups, alkyl carbonyl groups, or R3 and R4 represent an alkylene group or the following fragment (III) forming together with the atoms to which it is bound a cyclic imido structure:

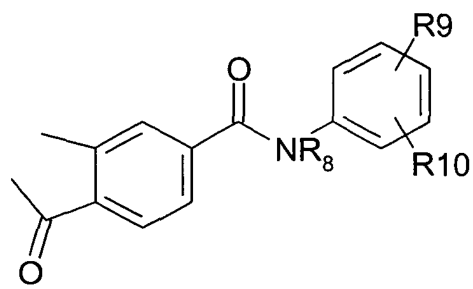

(III)

wherein R8 represents a hydrogen atom, or a $C_{1-4}$ alkyl group which may be substituted by one or more halogen atoms, or an acyl group; R9 and R10 which may be the same or different are selected from the group of halogen atoms, alkyl groups, cycloalkyl groups, alkenyl groups, alkinyl groups, aryl groups, aralkyl groups, hydroxyl group, alkoxy groups, amino group, aminoalkyl group, dialkylamino group, carboxyl group alkoxy carbonyl groups, alkylamino carbonyl groups, dialkylamino carbonyl groups, alkyl carbonyl groups.

The present invention also provides a method of producing a computer readable database comprising a representation of a compound as defined above capable of binding amino acids of a binding pocket of a Gβγ protein by noncovalent bonds, said method comprising

(a) introducing into a computer program a computer readable database comprising the threedimensional molecular structural coordinates of a binding pocket of a Gβγ protein for a compound as defined above, whereby the database is obtainable by

  (a1) obtaining threedimensional structural coordinates defining said protein or the binding pocket of said protein, from a crystal of said protein or co-crystal of said protein in a complex with phosducin, and

  (a-2) introducing said structural coordinates into a computer to produce a database containing the molecular structural coordinates of said protein or said binding pocket;

(b) generating a threedimensional representation of a binding pocket of said Gβγ protein in said computer program;

(c) superimposing at least one compound as defined above on said representation of the binding pocket;

(d) assessing the binding of the compound in the binding pocket;
(e) storing a representation of the compound into a computer readable database.

**Brief description of the Figures**

**Figure 1:** Inhibition of Gβγ-dependent GRK-2 phosphorylation of rhodopsin by the compounds. GRK-2: 20nM, rhodopsin: 400 nM, Gβγ 10 nM for COM1, 20 nM for COM2 and COM3. The compounds were added to the assay in concentrations ranging from 0.03 to 10μM. GRK-2-mediated phosphorylation of rhodopsin was inhibited by all compounds.

**Figure 2:** The compounds show no effect on Gβγ-independent phosphorylation of tubulin-β3 by GRK-2 at physiological concentrations: 2: 50 nM, tubulin: 3 μM for COM1 and 400 nM for COM2 and 3. The compounds were present in the assay at increasing concentrations, ranging from 0.1 - 100 μM.

**Figure 3:** Effect of increasing concentrations of Gβγ on the inhibition of Gβγ-dependent GRK-2-mediated phosphorylation of rhodopsin by COM2. GRK-2: 50nM, rhodopsin: 100nM, COM2: 3μM, Gβγ was present in the assay in concentrations ranging from 3 to 1000 nM. As a control, the same experiment was performed without COM2. The inhibitory effect of COM2 on GRK-2 activity is completely reversed at a Gβγ concentration of 1000 nM.

**Figure 4:** Inhibition of Gβγ-dependent GRK-2 activity by COM1 can be reversed by increasing the concentrations of its substrates rhodopsin. GRK-2: 50 nM, COM1: 30 μM, rhodopsin: 10-3000 nM.

**Figure 5:** Effects of COM 2 and 3 on inositol phosphate formation in CHO alpha2A receptor cells. The cells were stimulated for 30 sec with 10 μM UK14304; the formation of inositol phosphates was measured using the ALPHA screen™ assay technology (upper panel). Additionally, the influence of the compounds on cell viability was tested under the same conditions as in the inositol assays with a WST-1 cell-viability assay (lower panel).

**Figure 6:** Specificity of calcium signalling in alpha 2A receptor-overexpressing CHO cells after stimulation with 1 μM UK14304. As a negative control, native CHO-FRT cells were stimulated with the same agonist. The calcium signal after agonist injection was only present in receptor-expressing cells.

**Figure 7 A:** Intracellular calcium release upon stimulation of the alpha2A receptor expressed in CHO FRT cells with 1 μM UK14304 after 30 minutes of incubation with 30 μM of either COM2 or COM3 versus buffer incubated cells. Emission curves: The calcium concentration is reflected by the ratio of the emission values at 510 nm of the two different excitation wavelenghths 340 and 380 nm. The curves are blank-corrected by substraction of a buffer injection curve. Replicates of 6 independent experiments are shown. B: The difference between the maximum value of the emission curve and the basal value before agonist injection is shown for different concentrations of COM2 compared to controls. n=8; p>0.05

**Figure 8 A:** Intracellular calcium release upon stimulation of the native M1 receptor in HEK 293 cells with 100 μM carbachol, measured after 30 minutes of pre-incubation with 30 μM of either COM2 or COM3 versus buffer incubated cells. Emission curves: The calcium concentration is reflected by the ratio of the emission values at 510 nm of the two different excitation wavelenghth 340 and 380 nm. The curves are blank-corrected by substraction of a buffer injection curve. Replicates of 3 independent experiments are shown. B: The difference between the maximum value of the emission curve and the basal value before agonist injection is shown for different concentrations of COM2 compared to controls. n=3; p>0.05

**Figure 9:** Effects of COM1-3 on cyclic AMP formation in single isolated rabbit cardiomyocytes. After 30 minutes preincubation with 10μM of each compound, myocytes were stimulated with 10 μM isoproterenol for 30min. cAMP formation was measured using the ALPHA screen™ assay technology (upper panel). Additionally, the influence of the compounds on cell viability was tested under the same conditions as in the other assays using a WST-1 cell-viability assay (lower panel).

**Figure 10:** Effect of COM1 on the contraction amplitude of calcium tolerant isolated rabbit cardiomyocytes. Following the measurement of basal contraction of each cell, the myocytes were perfused with 10 μM COM1 for 10 minutes. Then, the contraction amplitude was monitored while aplying increasing concentrations of the β-receptor

agonist isoproterenol ($1x10^{-9}$ - $1x10^{-7}$M) in the presence of 10 μM COM1. A group of control cells was subjected to the same protocol, but COM1 was omitted from the perfusion medium. n = 8 cells were investigated in each group. The measurement of the velocity of cell shortening (corresponding to contractility in vivo) resulted in the same effects (not shown).

**Figure 11:** Binding interaction between a preferred binding pocket of Gβγ and COM2. The binding pocket includes amino acids βR42, βQ44, βM45, βR46, βT47, βV307, βL308, βG310, βW339.

**Figure 12:** Binding interaction between a preferred binding pocket and COM3.The binding pocket includes amino acids βR42, βQ44, βM45, βR304, βV307, βA309, βW339.

**Figure 13:** Cartesian coordinates of heavy atoms of a preferred binding pocket based on crystallographic data in The Protein Database format.

## Detailed description of the invention

The compounds according to the present invention are represented by formula (I). Preferably, the compounds of the invention have a molecular weight of less than 1500, preferably less than 1000.

In the formula, an aromatic carbocyclic ring includes aromatic substituents having 6 to 14 carbon atoms, preferably 6 to 10 carbon atoms. Specific examples are phenyl, naphtyl, anthracenyl, and phenanthryl whereby phenyl is preferred. An aromatic heterocyclic ring includes heteroaromatic five or six-membered cyclic moieties having 1 to 3 heteroatoms selected from nitrogen, oxygen, and sulfur such as electron rich heterocylic rings, for example pyrrolyl, pyrazolyl, imidazolyl, furanyl, thiophenyl, oxazolyl, isoxazolyl, thiazolyl, isothiazolyl, triazolyl and tetrazolyl, or electron deficient heterocyclic rings such as pyridyl or pyrimidyl, pyridazinyl, pyrimidinyl, pyrazinyl. The aromatic heterocyclic ring also includes benzoanellated heteroaromatic five or six-membered rings such as benzo[b]pyrrolyl (indolyl), benzo [c]pyrrolyl (isoindolyl), benzo[b]furanyl, and benzo[c]thiophenyl, quinolinyl, isoquinolinyl, cinnolinyl, phtalazinyl, quinazolinyl, and quinoxalinyl.

In the formula, e.g. as substituents R5, R6, R9 or R10, an alkyl group can include linear or branched alkyl groups having 1 to 6 carbon atoms, preferably 1 to 4 carbon atoms, for example, methyl, ethyl, n-propyl, isopropyl, n-butyl, isobutyl, sec-butyl, tert-butyl, n-pentyl, isopentyl and n-hexyl. A $C_{1-4}$ alkyl group is for example methyl, ethyl, n-propyl, isopropyl, n-butyl, isobutyl, sec-butyl or tert-butyl.

Examples of the halogen atoms include fluorine chlorine, bromine and iodine.

Examples of the alkenyl group can include linear or branched alkenyl groups having 2 to 6 carbon atoms, preferably 2 to 4 carbon atoms and 1 or 2 double bonds, for example, vinyl, propenyl, butenyl, isobutenyl and butadienyl.

Examples of the alkinyl group can include linear or branched alkinyl groups having 2 to 6 carbon atoms, preferably 2 to 4 carbon atoms and 1 or 2 triple bonds, for example, ethinyl, propinyl, and butinyl.

Examples of the cycloalkyl group can include those having 3 to 6 carbon atoms, for example, cyclopropyl, cyclobutyl, cyclopentyl and cyclohexyl.

Examples for an alkoxy group can include linear or branched alkoxy groups having 1 to 6 carbon atoms, preferably 1 to 4 carbon atoms, for example, methoxy, ethoxy, n-propoxy, isopropoxy, n-butoxy, isobutoxy, sec-butoxy, tert-butoxy, n-pentoxy, isopentoxy and n-hexoxy. Examples for the alkoxy carbonyl group can include a carbonyl group substituted by one of the above alkoxy groups.

Examples of the cycloalkyloxy group can include those having 3 to 6 carbon atoms, for example, cyclopropyloxy, cyclobutyloxy, cyclopentyloxy and cyclohexyloxy.

In the formulae, illustrative of the aryl group can be phenyl, naphthyl and pyridyl, with phenyl and pyridyl being particularly preferred. An aralkyl group is an alkyl group as defined above which is substituted by an aryl group.

Examples of the alkylene group can be a linear or branched one having 1 to 6 carbon atoms, with one having 2 to 4 carbon atoms being preferred. Illustrative can be methylene, ethylene, and trimethylene.

Examples for an alkylamino group can include an amino group having a substituent selected from linear or branched alkyl groups having 1 to 6 carbon atoms, preferably 1 to 4 carbon atoms, for example, methyl, ethyl, n-propyl, isopropyl, n-butyl, isobutyl, sec-butyl, tert-butyl, n-pentyl, isopentyl and n-hexyl. Examples for the alkylamino carbonyl group can include a carbonyl group substituted by one of the above alkylamino groups.

Examples for a dialkylamino group can include an amino group having two substituents selected from linear or branched alkyl groups having 1 to 6 carbon atoms, preferably 1 to 4 carbon atoms, for example, methyl, ethyl, n-propyl, isopropyl, n-butyl, isobutyl, sec-butyl, tert-butyl, n-pentyl, isopentyl and n-hexyl. Examples for the dialkylamino carbonyl group can include a carbonyl group substituted by one of the above dialkylamino groups.

Examples of the cycloalkylamino group can include those having 3 to 6 carbon atoms, for example, cyclopropylamino, cyclobutylamino, cyclopentylamino and cyclohexylamino.

Examples of the acyl group can include acyl groups having 1 to 7 carbon atoms, preferably 2 to 5 carbon atoms, for example formyl, acetyl, propionyl.

The carboxyl group may be in the form of a pharmaceutically acceptable metal salt, such as an alkali metal salt or an alkaline earth metal salt.

The above groups, in particular the aryl and alkyl groups, may contain 1 to 3 substituents. Examples of such substituents can include halogen atoms, $C_{1-4}$ alkyl groups, $C_{1-4}$ alkoxy groups, $C_{1-4}$ alkylthio groups, $C_{1-4}$ alkylsulfinyl groups, $C_{1-4}$ alkylsulfonyl groups, carboxyl group, $C_{2-5}$ alkoxycarbonyl groups, nitro group, amino group, and $C_{1-4}$ alkylamino groups. Here, illustrative of the halogen atoms can be fluorine, chlorine, bromine and iodine. The $C_{1-4}$ alkyl groups are, for example, methyl, ethyl, n-propyl, isopropyl and n butyl. Illustrative of the $C_{1-4}$ alkoxy groups are, for example, methoxy, ethoxy and propoxy. Illustrative of the $C_{1-4}$ alkylthio groups are, for example, methylthio, ethylthio and propylthio. Illustrative of the $C_{1-4}$ alkylsulfinyl groups are for example, methylsulfinyl, ethylsulfinyl and propylsulfinyl. Illustrative of the $C_{1-4}$ alkylsulfonyl groups are, for example, methylsulfonyl, ethylsulfonyl and propylsulfonyl. Illustrative of the $C_{2-5}$ alkoxycarbonyl groups can be those having alkoxy groups each of which contains 1 to 4 carbon atoms, for example, methoxycarbonyl, ethoxy carbonyl and propoxycarbonyl. Illustrative of the $C_{1-8}$ alkylamino groups can be those having one or two alkyl groups each of which contains 1 to 4 carbon atoms, for example, methylamino, dimethylamino, ethyl amino and propylamino. The alkyl moieties in these substituents may be linear, branched or cyclic.

The compounds of the invention may also contain common protecting groups for functional groups such as carboxyl groups. Moreover, the compounds of the invention may also be in the form of a prodrug which may be converted to an active agent under physiological conditions. As an Example, a carboxyl group containing compound of the invention may be converted into a pharmaceutically acceptable derivative such as an ester, amide, anhydride or ether, for improving pharmacological properties whereby the pharmaceutically acceptable derivative is hydrolysed under physiological conditions whereby a compound of the present invention is generated.

The compounds of the invention are characterised by a linear chain of rigid segments containing specific aromatic moieties spacing apart at least two amide or imide linking groups. Moreover, the compounds of the invention are further characterised by substituents capable of forming non-covalent bonds, such as hydrogen bonds, for example with amino acid residues of a specific binding pocket of Gβγ. The compounds of the invention interact with the Gβγ protein complex, thereby triggering an effect *in vivo* similar to the effect observed due to the interaction of phosducin with the Gβγ protein complex.

In general formula (II), X represents -CONR3-, -NR3CO-, or -NR3COCH=CH-, whereby -NR3CO-, or -NR3COCH=CH- are preferred.

In general formula (I), L represents a single bond, an oxygen atom, a sulfur atom, a $CR^5R^6$ group or a $NR^5$ group wherein $R^5$ and $R^6$ are selected from a hydrogen atom, an alkyl group and a halogen atom. Preferably, L is a single bond, an oxygen atom or a difluoromethylene group.

In general formula (II), R1 and R2 which may be the same or different represent a hydrogen atom, a carboxyl group, a carboxamide group, a group COOR7, wherein R7 represents an alkyl group, an aryl group, or an aralkyl group. Preferably, when L is a single bond, at least one R1 is a a carboxyl group, or a carboxamide group and R2 is a hydrogen atom.

In general formula (II), R3 may represent a hydrogen atom, an acyl group, an alkyl group which may be substituted by a halogen atom. Preferably, R3 is a hydrogen atom.

In general formula (II), R4 represent an aromatic carbocyclic or heterocyclic ring, which may be substituted by one or more substituents selected from the group of halogen atoms, alkyl groups, cycloalkyl groups, hydroxyl group, alkoxy groups, cycloalkoxy groups, amino group, alkylamino group, dialkylamino group, cycloalkylamino group, carboxyl group, alkoxy carbonyl groups, alkylamino carbonyl groups, dialkylamino carbonyl groups, and acyl groups. Preferably, R4 is an optionally substituted 5- or 6-membered ring.

In general formula (II), R3 and R4 may represent together, in particular when L is not a single bond, an alkylene group or the following fragment (III) forming together with the atoms to which it is bound a cyclic structure:

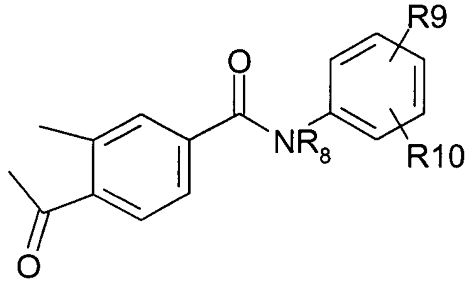

(III)

wherein R8 represents a hydrogen atom, or an alkyl group which may be substituted by one or more halogen atoms, or an acyl group; R9 and R10 which may be the same or different are selected from the group of halogen atoms, alkyl groups, cycloalkyl groups, alkenyl groups, alkinyl groups, aryl goups, aralkyl groups, hydroxyl group, alkoxy groups, amino group, aminoalkyl group, dialkylamino group, carboxyl group, carboxamido group, alkoxy carbonyl groups, alkylamino carbonyl groups, dialkylamino carbonyl groups, or acyl groups. In fragment (III) R8 is preferably a hydrogen atom. Fragment (III) is preferably present in case L is not a single bond whereby preferably one of R9 and R10 is a hydrogen atom and the other of R9 and R10 is different from a hydrogen atom, preferably a carboxyl group or carboxamido group in ortho position to the amide bond.

It is preferred that A and B are the same groups of formula (II) whereby the compounds contain a plane of symmetry. A preferred class of compounds contains embodiments wherein L is a single bond. In this case, compounds of formula (I) are biphenyl derivatives. Preferably, the biphenyl moiety of the molecule contains groups contributing to the hydrophilicity of the molecule, such as carboxyl groups, whereby at least one of R1 and R2 does not represent a hydrogen atom. In case of a biphenyl derivative, X is preferably -NR3CO-.

In a preferred embodiment, R3 is a hydrogen atom and A and B are independently represented by the following formula (IV)

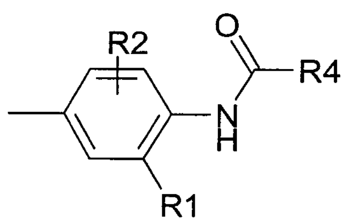

(IV)

wherein R1, R2 and R4 are as defined above.

A preferred class of compounds of the present invention are represented by the following formula (V)

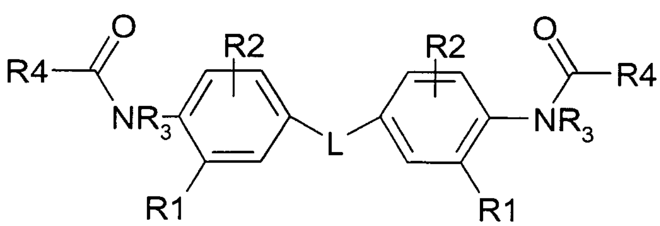

wherein L, R1, R2, R3, and R4 are a s defined above.

A further preferred class of compounds of the present invention are represented by the following formula (VI)

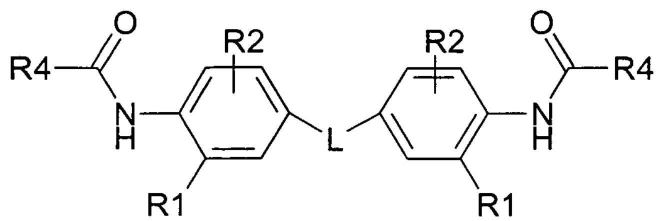

wherein L, R1, R2, and R4 are a s defined above.

A further preferred class of compounds of the present invention are represented by the following formula (VII)

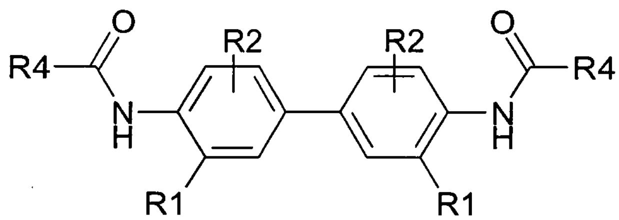

wherein R1, R2, and R4 are a s defined above.

A further preferred class of compounds of formula (I) contains embodiments wherein L is an oxygen atom or a $CF_2$ group. In this case, it is preferred that R3 and R4 represent the above fragment (III) forming together with the atoms to which it is bound a cyclic imido structure, wherein R8, R9 and R10 are as defined above.

The most preferred componds of the present invention are represented by the following formulae.

Compound 1 (COM 1) 4,4'-bis[(4-fluorobenzoyl)amino][1,1'-biphenyl]-3,3'-dicarboxylic acid

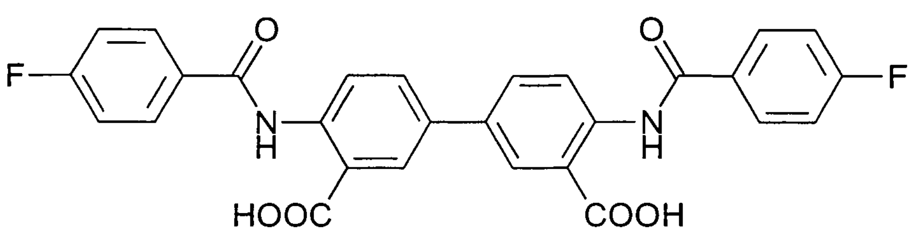

Compound 2 (COM 2) 2-[({2-[4-(4-{5-[(2-carboxyanilino)carbonyl]-1,3-dioxo-1,3-dihydro-2H-isoindol-2-yl}phenoxy) phenyl]-1,3-dioxo-2,3-dihydro-1H-isoindol-5-yl}carbonyl)amino]benzoic acid

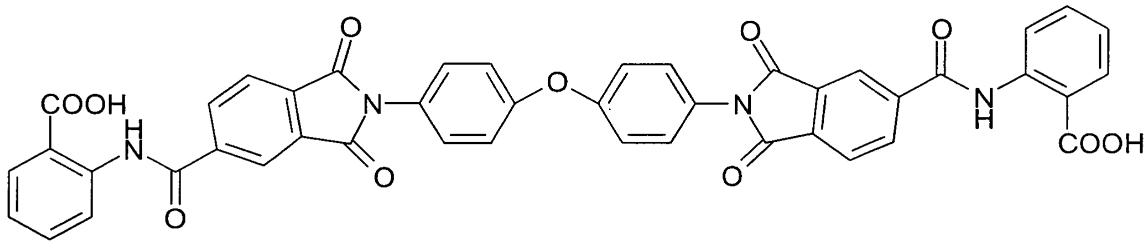

Compound 3 (COM 3) 4,4'-bis{[3-(2-furyl)acryloyl]amino}[1,1'-biphenyl]-3,3'-dicarboxylic acid

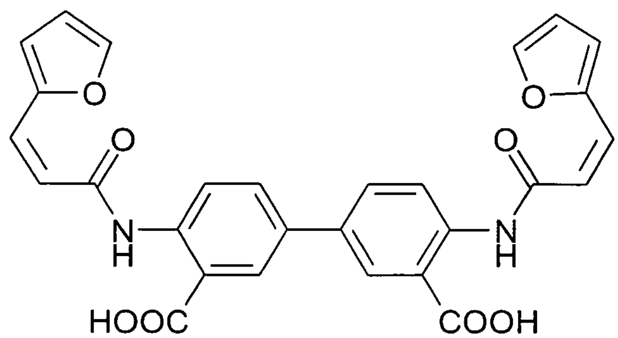

No particular limitation is imposed on the salt of the compunds of the present invention, said salt also pertaining to the present invention, insofar as it is a pharmacologically acceptable salt. Illustrative can be alkaline metal salts such as sodium or potassium salts, alkaline earth metal salts calcium and magnesium salts, or salts containing organic cationic ions such as ammonium ions. Illustrative can also be acid addition salts of mineral acids, such as the hydrochloride, hydrobromide, hydroiodide, sulfate, nitrate and phosphate; and acid addition salts of organic acids, such as the benzoate, methanesulfonate, ethane-sulfonate, benzenesulfonate, p-toluenesulfonate, oxalate, maleate, fumarate, tartrate and citrate.

Further, the compounds according to the present invention may exist in the form of solvates represented by hydrates. Further, the compounds according to the present invention may exist as geometric isomers. Such geometric isomers should also be encompassed by the present invention. Further, the compounds according to formulae (I) and (II) are optically active and exist in the form enantiomers. Such enantiomers may be obtained in pure form accdording to conventional resolution methods and should also be encompassed by the present invention.

Some of the compounds are covered by the present claims are commercially available from different sources. For example 4,4'-bis[(4-fluorobemzoyl)amino][1,1'-biphenyl]-3,3'-dicarboxylic acid (COM1), 2-[({2-[4-(4-{5-[(2-carboxyanilino)carbonyl]-1,3-dioxo-1,3-dihydro-2H-isoindol-2-yl}phenoxy)phenyl]-1,3-dioxo-2,3-dihydro-1H-isoindol-5-yl}carbonyl)amino]benzoic acid (COM2), and 4,4'-bis{[3-(2-furyl)acryloyl]amino}[1,1'-biphenyl]-3,3'-dicarboxylic acid (COM3) are known compounds and commercially available. The compounds according to the present invention can be prepared according to known methods, for example, by the following processes. Numbers given in square brackets below are CAS-numbers.

Symmetrical compounds of general formula (I) wherein X is -NR3CO- or -NR3COCH=CH- may be prepared by acylation of a corresponding diamine (X) by an acylating agent (XI) according to the following scheme 1 wherein LG represents a leaving group and R1, R2, R3, R4, and L are as defined above:

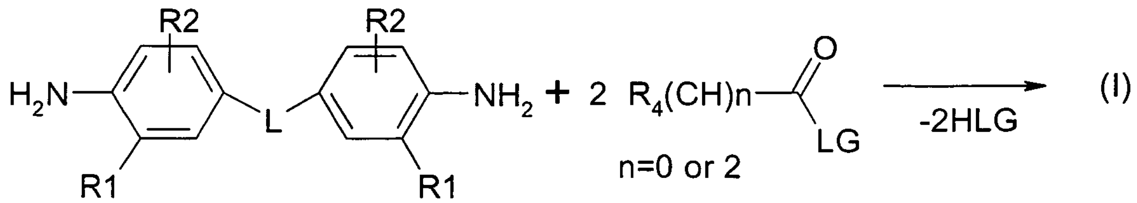


(X) (XI)

Accordingly, one mole of compound (X) is reacted in a suitable solvent, preferably in the presence of a base, with two moles of compound (XI) at a temperature from -10°C to the boiling point of the solvent. The leaving group is not particularly limited and can be any suitable leaving group such as a halogen atom. The reaction conditions are not particularity limited and can be adopted from the following literature references: Beckwith; in Zabicky *The Chemistry of Amides*; Wiley: New York, 1970, pages 73 to 185. Since the reaction is usually highly exothermic, it must be carefully controlled by cooling or dilution in a suitable solvent. Acylating compound (XI) may be prepared from the corresponding caboxylic acid. Usually, compound (XI) is a carboxylic acid chloride which may be prepared by the reaction of the corresponding carboxylic acid with thionyl chloride or phosphoroous pentachloride. When milder conditions are necessary due to the presence of acid-sensitive functional groups, then the acid chloride may be prepared by reacting the corresponding acid with oxalyl chloride, usually in a hydrocarbon solvent, under essentially neutral conitions. Alternatively, the amide bond may be formed by generating an acyl halide *in situ* by refluxing two moles the corresponding carboxylic acid, triphenylphosphine, bromotrichloromethane and one mole of the compound of formula (X) to give the desired compound of formula (I), Barstow *et al*. J. Org. Chem. **36,** 1305, (1971). Alternatively, LG may be an imidazoline moiety whereby the compound of formuly (XI) may prepared according to Staab *et al*., Newer Methods Prep. Org. Chem. **5,** 61, (1968). Furthermore, the corresponding carboxylic acid of compound (XI) may be converted to the acylating agent (XI) by cyclohexylcarbodiimide, Kurzer *et al*., Chem. Rev. **67,** 107, (1967).

A large number of biaryl starting compounds (X) are known compounds such as benzidine, and 3, 3'-dimethylbenzidine [119-93-7] which can be converted to the corresponding diacid by oxidation with an oxide of manganese or chromium, Clarke *et al*, Org. Synth. **II**, 135 (1943), Friedmann, Org. Synth. **43,** 80 (1963); J. Org. Chem. **30, 1453** (1965). Other biaryl starting compounds (X) may be prepared by an Ullmann reaction, i.e. the dehalogen-coupling of aryl halides with copper, cf. Fanta *Synthesis* (1974), 9-21; Bringmann; Walter; Weirich *Angew. Chem. Int. Ed. Engl.* (1990), 29, 977-991; Sainsbury *Tetrahedron* (1980), 36, 3327-3359. A preferred aryl halogenide is an aryl iodide. Alternatively, the biaryl starting compounds may be prepared by a Suzuki reaction, ie the palladium-catalyzed cross coupling of organic halides or perfluorinated sulfonates with organoboron derivatives, N. Miyaura et al., Tetrahedron Letters 1979, 3437; N. Miyaura, A. Suzuki, Chem. Commun. 1979, 866; A. Suzuki, Pure Appl. Chem. 63, 419-422 (1991); A. R. Martin, Y. Yang, Acta Chem. Scand. 47, 221-230 (1993).

A large number of diphenyl ether starting compounds (X) are known compounds such as bis-(4-aminophenyl) ether [101-80-4]. Other diphenyl ether starting compounds may be prepared according to conventional methods known in the art. A large number of diphenyl methane starting compounds (X) are known compounds such as bis-(4-aminophenyl)methane [101-77-9]. Other diphenyl methane starting compounds may be prepared according to conventional methods known in the art.

Symmetrical compounds of formula (I) wherein X is -CONR3- or may be prepared according to the following scheme 2 by acylation of a corresponding amine (XIII) by an acylating agent (XII) according to the following scheme 2 wherein LG represents a leaving group and R1, R2, R3, and R4 and L are as defined above.

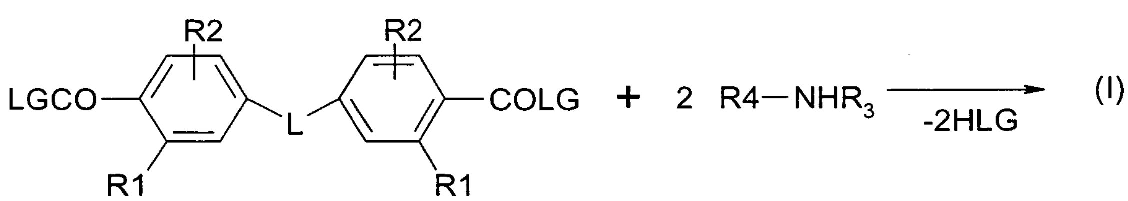


(XII) (XIII)

Accordingly, one mole of compound (XII) is reacted in a suitable solvent, preferably in the presence of a base with two moles of compound (XIII) at a temperature from -10°C to the boiling point of the solvent.The reaction condition are not particularily limited and can be adopted from the following literature reference: Beckwith; in Zabicky *The Chemistry ofAmides*; Wiley: New York, 1970, pages 73 to 185 and the literature referred to above regarding scheme 1.

A large number of biaryl starting compounds (XII) are known compounds such as biphenyl-4,4'-dicarboxylic acid [787-70-2] which can be converted to the corresponding acid chloride by reaction with thionyl chloride. Other biaryl starting compounds may be prepared accrding to conventional methods, e.g. by coupling suitably substituted and optionally protected aryl chlorides in an Ullmann reaction with a copper catalyst or by a palladium-catalyzed cross coupling of an optionally protected aryl halide or with arylboron derivatives in a Suzuki reaction.

In case of a compound of formula (I) wherein R3 and R4 form together a moiety of formula (III), the compound may be prepared according to the following scheme 3 wherein R1, R2, R8, R9, and R10 are as defined above :

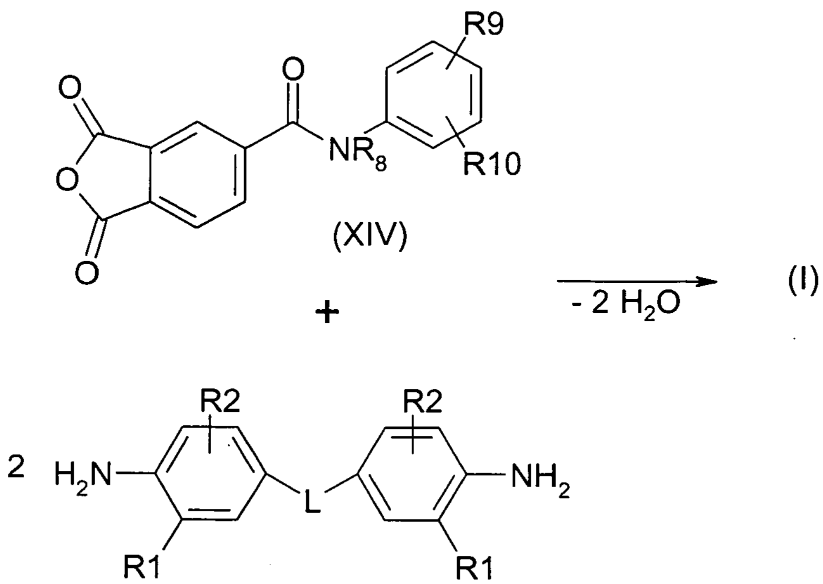


(XIV) + 2 (XV) → (I)

(XV)

Accordingly, one mole of compound (XIV) is reacted in a suitable solvent, preferably in the presence of a base with two moles of compound (XV) at a temperature from -10°C to the boiling point of the solvent, optionally at an elevated pressure.

Unsymmetrical biphenyl compounds of general formula (I) may be prepared by coupling a suitable organoboron derivative (XII) with a suitable organic halide or perfluorinated sulfonate according to the following scheme 4:

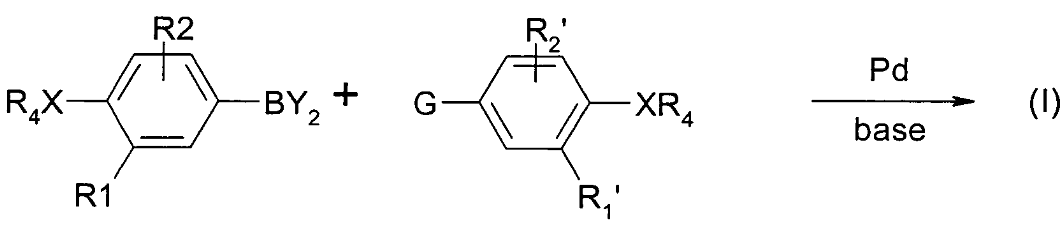


(XVI) (XVII)

wherein R1, R2, R4 and X are as defined above and BY2 represents B(OR)2 wherein R is an alkyl group, 9-BBN, or $B(CHCH_3CH(CH_3)_2)_2$; G is I, Br, Cl or $OSO_2(C_nF_{2n+1}$, n=0,1,4) such as triflate, and Pd is a palladium catalyst such as tetrakistriphenylphosphine palladium (0) or $Pd(dppf)Cl_2$. Suitable bases are sodium carbonate, NaOEt, TIOH, triethylamine or potassium phosphate. N. Miyaura et al., Tetrahedron Letters 1979, 3437; N. Miyaura, A. Suzuki, Chem. Commun. 1979, 866; A. Suzuki, Pure Appl. Chem. 63, 419-422 (1991); A. R. Martin, Y. Yang, Acta Chem. Scand. 47,

221-230 (1993).

Starting compound (XVI) may be prepared in two steps by conversion according to the following scheme 5:

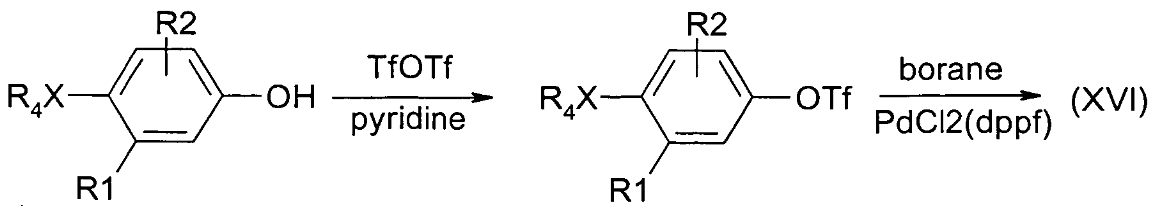


(XVIII) (XIX)

wherein R1, R2, R4 and X are as defined above and Tf means triflate. Accordingly, the corresponding phenol precursor (XVIII) is converted into the corresponding triflic acid ester (XIX) by treatmentwith triflic anhydride in a solvent such as pyridine at a temperature of between 0°C and the boiling point of the solvent, followed by a reaction of the triflic acid ester (XIX) thus obtained with an excess of a suitable borane such as pinacolborane or 9-BBN in a suitable solvent such as dioxane at a temperature of from 0°C to the boiling point of the solvent. If compound (XIX) and compound (XVI) are coupled, symmetrical compounds of the invention are obtained.

Starting compound (XVII) is the same intermediate compound (XIX) when G is a OTf, may be prepared as disclosed above.

In case G is a halogen atom (Hal) and X is -NR3CO- or -NR3COCH=CH-, starting compound (XVII) may be prepared according to the following scheme 6:

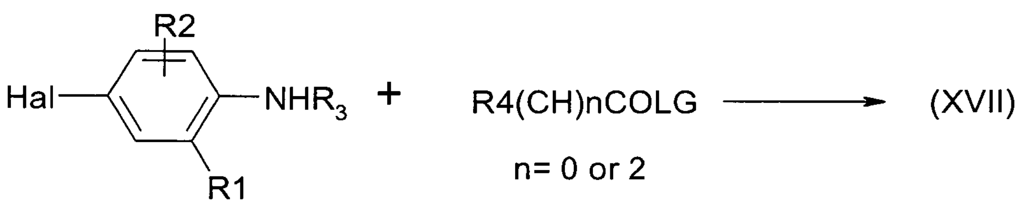


wherein Hal is a halogen atom such as Cl, Br, or I, and R1, R2, R3, and R4 are as defined above. Accordingly, one mole of a suitable amine is reacted in a suitable solvent, preferably in the presence of a base, with one mole of an acylating agent at a temperature from -10°C to the boiling point of the solvent. The leaving group is not particularly limited and can be any suitable leaving group such as a halogen atom. The reaction condition are not particularily limited and can be adopted from the following literature references: Beckwith; in Zabicky *The Chemistry of Amides*; Wiley: New York, 1970, pages 73 to 185 and the literature referred to above regarding scheme 1.. Since the reaction is usually highly exothermic, it must be carefully controlled by cooling or dilution in a suitable solvent.

In case G is a halogen atom (Hal) and X is -CONR3-, starting compound (XVII) may be prepared according to the following scheme 7:

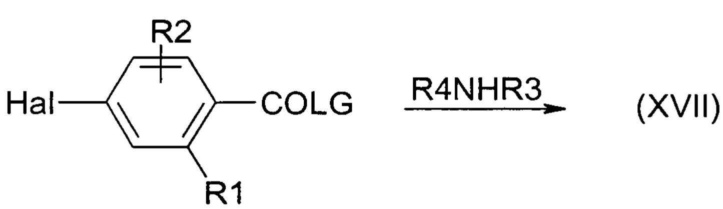


Accordingly, one mole of a suitable aromatic carboxylic acid derivative is reacted in a suitable solvent, preferably in the presence of a base with one mole of a suitable amine compound at a temperature from -10°C to the boiling point of the solvent.The reaction condition are not particularily limited and can be adopted from the following literature reference: Beckwith; in Zabicky *The Chemistry ofAmides;* Wiley: New York, 1970, pages 73 to 185 and the literature referred to above regarding scheme 1.

In case G is a halogen atom (Hal) and X is -NR3CO-, and R3 and R4 represent together fragment (III) forming together with the atoms to which it is bound a cyclic imido structure, starting compound (XVII) may be prepared according to the following scheme 8:

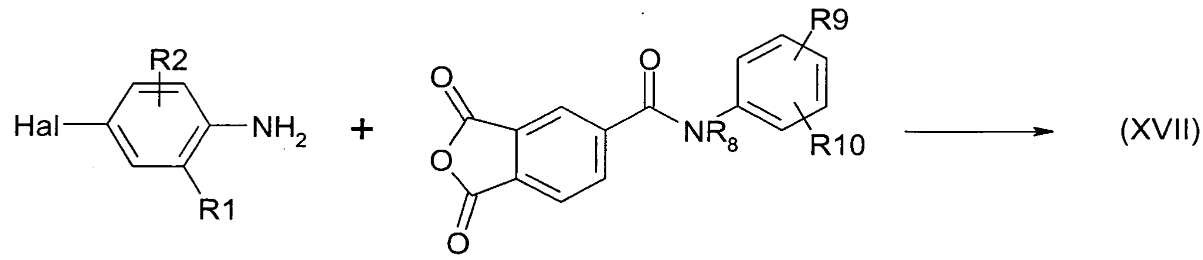

The above anhydride may be prepared for example departing from 1,2,4-benzene tricarboxylic acid anhydride chloride [1204-28-0] and a suitable amine compound according to the following scheme 9:

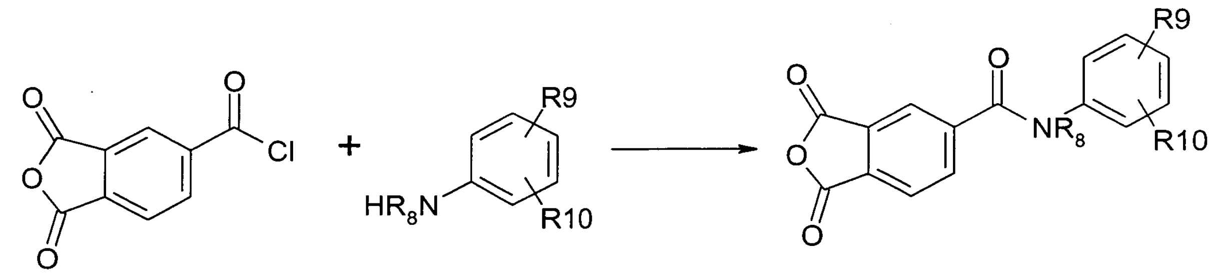

Separation and isolation of the compounds may be conducted by standard laboratory methods available to the skilled person. During preparation of the compounds of the invention, protective groups for functional groups may be required and can be used according to the general knowledge in the art.

The compounds of the invention may be used in the treatment of cardiovascular disease. Specific examples of cardiovascular diseases related to congestive heart failure are hypertension, myocardial ischemia, cardiomyopathy or congenital heart disease. The compounds of the invention are administered for increasing the contractility of muscle cells of the heart. Preferebly, the compound of the invention decrease IP3 of a heart muscle cell in response to a Gi coupled agonist. On the other hand, the compounds of the invention preferably hardly have an influence on cAMP in a heart muscle cell.

The compounds of formula (I) or pharmaceutically acceptable salts or esters thereof can be used as medicaments, e.g. in the form of pharmaceutical preparations. The pharmaceutical preparations can preferably be administered orally, e.g. in the form of tablets, coated tablets, dragees, hard and soft gelatine capsules, solutions, emulsions or suspensions. However, the administration can also be effected rectally, e.g. in the form of suppositories, or parenterally, e.g. in the form of injection solutions.

The present invention provides a pharmaceutical composition which comprises a compound of formula (I), in particular a preferred compound as described above, and a pharmaceutically acceptable carrier. The compounds of formula (I) and pharmaceutically acceptable salts or esters thereof can be processed with pharmaceutically acceptable carriers, e.g. inert, inorganic or organic carriers for the production of pharmaceutical preparations. Lactose, corn starch or derivatives thereof, talc, stearic acid or its salts and the like can be used, for example, as such carriers for tablets, coated tablets, dragees and hard gelatine capsules. Suitable carriers for soft gelatine capsules are, for example, vegetable oils, waxes, fats, semi-solid and liquid polyols and the like; depending on the nature of the active substance no carriers are, however, usually required in the case of soft gelatine capsules.

Suitable carriers for the production of solutions and syrups are, for example, water, polyols, sucrose, invert sugar, glucose and the like. Adjuvants, such as alcohols, polyols, glycerol, vegetable oils and the like, can be used for aqueous injection solutions of water-soluble salts of compounds of formula (I), but as a rule are not necessary. Suitable carriers for suppositories are, for example, natural or hardened oils, waxes, fats, semi-liquid or liquid polyols and the like.

In addition, the pharmaceutical preparations can contain preservatives, solubilizers, stabilizers, wetting agents, emulsifiers, sweeteners, colorants, flavorants, salts for varying the osmotic pressure, buffers, masking agents

or antioxidants. They can also contain still other therapeutically valuable substances.

Medicaments containing a compound of formula (I) or a pharmaceutically acceptable salt or ester thereof and a therapeutically inert excipient are also an object of the present invention, as is a process for the production of such medicaments which comprises bringing one or more compounds of formula (I) or pharmaceutically acceptable salts or esters thereof and, if desired, one or more other therapeutically valuable substances into a galenical dosage form together with one or more therapeutically inert carriers.

Accordingly, also part of this invention is a method of treating cardiovascular disease such as congestive heart failure whereby the method comprises administering to a patient having any of the above conditions an amount of the pharmaceutical composition of this invention effective to treat or prevent said condition.

The dosage can vary within wide limits and will, of course, be fitted to the individual requirements in each particular case. In general, the effective dosage for oral or parenteral administration is between 0.01-20 mg/kg/day, with a dosage of 0.1-10 mg/kg/day being preferred for all of the indications described. The daily dosage for an adult human being weighing 70 kg accordingly lies between 0.7-1400 mg per day, preferably between 7 and 700 mg per day.

Binding of a compound of the invention to Gβγ in myocardial cells, notably a binding site which is also a binding site for phosducin, is responsible for an increase in contractility and sensitivity of a heart, a heart muscle or cells of a heart muscle. Accordingly, the binding of the compounds of the invention may be considered in order to optimise the effect of drugs which can inhibit the function of Gβγ in myocardial cells by selecting antagonists of Gβγ signalling pathways for the treatment of congestive heart failure.

The selection of specific compounds of the invention which have specific properties of interaction with Gβγ in myocardial cells may be conducted by the principles of rational drug design based on the characteristics of the pharmacophore common to all compounds of formula (I) and the protein structure of Gβγ. A crystal structure at 2.4 Å resolution of the complex of Gβγ and phosducin has been published (Gaudet et al., Cell, 87, 577-588, (1996)) and the coordinates of the phosducin/Gβγ structure are available from the Protein Database (entries 1 AOR, 1 B9X, 1 B9Y, and 2TRC). Moreover, further crystal structures of Gβγ or the interaction of Gβγ and phosducin or an active N-terminal truncated phosducin are available according to general methods known in the art.

Computer-based methods of the invention may be divided into two classes: database methods and de novo design methods. In database methods according to the invention, a compound of formula (I) such as COM1, COM2 or COM3 is compared to all compounds present in a database of chemical representations of compounds of formula (I) and compounds whose structure is in a specific desired way similar to the compound such as COM1 COM2 or COM3 are identified. The representations in the database may be based on either experimental data, generated by NMR or x-ray crystallography, or modeled three-dimensional structures based on two-dimensional data. In de novo design methods of the invention, models of compounds of formula (I) whose structure is in a specific desired way similar to the compound of interest such as COM1, COM2 or COM3 are generated by a computer program using information derived from known structures, e.g., data generated by x-ray crystallography and/or theoretical rules. Such design methods can build a compound of formula (I) having a desired structure in either an atom-by-atom manner or by assembling stored small molecular fragments. Both database and de novo methods of the invention are based on the identification of the pharmacophore important for biological activity, as disclosed herein.

Based on the threedimensional structure of Gβγ, a compound of the present invention can be examined through the use of computer modelling using a standard docking program such as GRAM, DOCK, or AUTODOCK (Goodsell et al. (1990) Proteins: Structure, Function and Genetics, 8, 195-201; Kuntz et al. (1982) J. Mol. Biol. 161, 269-288). This procedure can include computer fitting of compounds of the invention to Gβγ. Computer methods can also be employed to estimate the attraction, repulsion, and steric hindrance of the compounds of the invention to a binding site of Gβγ. Generally, the tighter the fit (e.g., the lower the steric hindrance, and/or the greater the attractive force) the more potent the compound of the invention will be as a drug since these properties are consistent with a tighter binding constant. Furthermore, the higher the specificity of the compound of the invention, the more likely it is that the drug will not interfere with related proteins, thereby minimizing potential side-effects due to unwanted interactions with other proteins.

Initially, a compound corresponding to compound 1, compound 2, or compound 3 belonging to the class of compounds of formula (I) or a preferred class as defined above may be systematically modified within the general class of formula (I) or a preferred class as defined above, by computer modeling programs until one or more desired bonding characteristics are identified. In a preferred embodiment of the method, assessing of the compound comprises fitting the compound to said representation and performing energy minimisation. Alternatively, compounds may be selected from the general class of compounds of formula (I) by judging whether or not characteristics such as types of functional groups and their relative three-dimensional positions correspond to those in a COM1, COM2, or COM3 already known to bind to the binding pocket of Gβγ (for example, a method utilizing programs such as ISIS-3D:MDL Information Systems, Inc.; UNITY: Tripos, Inc.; and Chem-X: Chemical Design Ltd). Based on the three-dimensional coordinates of a binding pocket of Gβγ, database search methods excluding any hypotheses and preconceptions can be utilized. Method for automatically estimating the most stable complex structure of a protein and a ligand are disclosed

in PCT International Publication WO93/20525; Yamada, M. et al., J. Mol. Biol., 243, pp.310-326, 1994, and, based on this method, a method for identifying ligand compounds capable of stably binding to the ligand-binding region of a target biopolymer from a three-dimensional structure database are available from PCT International Publication WO96/13785; the 24th Symposium for Structure-Activity Relationship, Mizutani M et al., subject number 14S20, 1996). According to this method a high speed evaluation of compounds of formula (I) from a database containing representations of two or more compounds based on energetic stability of complexes is possible. Departing from the basic molecular skeleton of the compounds of formula (I), a high-speed database search method based on the matching of topologies can also be utilized according to PCT/JP96/03325. Programs suitable for generating predicted three-dimensional structures from two-dimensional data include: Concord (Tripos Associated, St. Louis, Mo.), 3-D Builder (Chemical Design Ltd., Oxford, U.K.), Catalyst (Bio-CAD Corp., Mountain View, Calif.), and Daylight (Abbott Laboratories, Abbott Park, III.). Programs suitable for searching three-dimensional databases to identify molecules bearing the desired pharmacophore of the compounds of formula (I) include: MACCS-3D and ISIS/3D (Molecular Design Ltd., San Leandro, Calif.), ChemDBS-3D (Chemical Design Ltd., Oxford, U.K.), and Sybyl/3DB Unity (Tripos Associates, St. Louis, Mo.).

A representation of the corresponding compound may then be stored in a database, Lam et al., Science 263: 380-384 (1994); Wlodawer et al., Ann. Rev. Biochem. 62:543-585 (1993); Appelt, Perspectives in Drug Discovery and Design 1:23-48 (1993). Such computer modeling allows the selection of a number of rational chemical modifications, as opposed to the countless number of essentially random chemical modifications that could be made within the general class of compounds of formula (I). Thus, by using three-dimensional structural analysis and computer modeling, a large number of these compounds can be rapidly considered computationally without laborious synthesis, and compounds of the formula (I) can be determined, which show desirable bonding properties. Selection criteria for the storage of the compound in the database may be based on the stability of the most stable docking structure between a compound of formula (I) and a binding pocket of Gβγ . In order to find the most stable docking structure between a given biopolymer and a ligand molecule, it is necessary to estimate stabilities of all possible binding modes (corresponding to rotation or translation of one molecule while fixing another compound), and possible conformations of compounds of formula (I). In a preferred embodiment the representations of the compounds stored in a database have a most stable docking structure which is more stable than a most stable docking structure of a reference compound, such as the docking structure of COM1, COM2 or COM3. The stability may be expressed by the bonding energy (including hydrogen bonding and van der Vaal's contributions) per 100 Daltons of the molecular weight of the compound of formula (I).

In the method of the invention, the binding pocket preferably is defined by the positions of the atoms of two or more, preferably five or more amino acids of the threedimensional structure of Gβγ defined by the partial sequences of from βR42 to βT47 and βR304 to βW339 of the β chain of Gβγ. Preferably, the binding pocket includes the threedimensional structure of Gβγ defined by the atoms of partial sequences of from βR42 to βT47, βR304 to βG310, and βW339. In a preferred embodiment, the binding pocket includes βR42, βQ44, βM45, βR46, βT47, βV307, βL308, βG310, βW339. In a further preferred embodiment, the binding pocket includes amino acids βR42, βQ44, βM45, βR304, βV307, βA309, βW339. One or more amino acids of the binding pocket bind to a compound of the invention by hydrogen bonds and/or dispersion forces.

The present invention also relates to a computer readable database produced according to the method of the invention and a machine readable medium embedded with the database of the invention.

Once one or more compounds are selected based on the binding properties to a binding pocket of Gβγ and a representation thereof is stored in a database, the one or more compounds can be either selected from a commercially available library of chemicals as are commercially available from most large chemical companies. The de novo synthesis of one or even a relatively small group of specific compounds is reasonable in the art of drug design and cannot be considered as an undue burden on the way to an active agent. Accordingly, in a preferred embodiment of the invention, the method further comprises

(f) preparing a compound represented in said computer readable database;
(g) contacting said compound in a binding assay with a Gβγ protein and
(h) determining whether the compound binds to the protein in the assay; and
(i) introducing a representation of the compound that has predetermined binding properties in said assay into a computer readable database.

The present invention also relates to a compound according to formuly (I) whose representation is contained in the computer readable database, and which is preferably further characterised by inhibiting the Gβγ-dependent activity of G protein-coupled receptor kinase (GRK-2) by more than 25 % in a rhodosin phosphorylation assay comprising the following steps:

(a) adding rhodopsin to a mixture containing Gβγ , GRK-2, ATP, and the compound;

(b) measuring the phosphorylation activity; and

(c) comparing the phosphorylation activity measured in step (b) with the phosphorylation activity of a mixture according to step (a) which does not contain the compound.

The compounds of the invention preferably decrease the intracellular calcium concentration of a heart muscle cell and IP3 of a heart muscle cell in response to $G_i$ coupled agonists. On the other hand, a compound of the invention does essentially not have an influence on cAMP in a heart muscle cell.

The medicine containing a compound of the invention can then be further optimised regarding the dosage in a competitive binding assay (including in high throughput binding assays) for its ability to bind to Gβγ in the presence of an active N-terminal truncated phosducin of the invention. Alternatively the dosage of the compound of the invention can be optimised by considering (1) its ability to increase the contractility of muscle cells in an assay; or (2) its ability to inhibit Gβγ-mediated processes. An assay may comprise the following steps:

(a) incubating a mixture comprising a predetermined concentration of Gβγ and a predetermined concentration of COM1, COM2, COM3, phosducin, an N-terminal truncated phosducin, or a function conservative variant under conditions which allow for binding of the COM1, COM2, COM3, phosducin, the N-terminal truncated phosducin, or the function conservative variant to Gβγ,

(b) incubating a mixture according to (a) under conditions according to (a) in the presence of a predetermined concentration of a test compound capable of binding to Gβγ, and

(c) selecting a test compound providing a higher concentration of COM1, COM2, COM3, phosducin, of said N-terminal truncated phosducin or of said variant not bound to Gβγ in the mixture of step (b) than the mixture of step (a).

In general, such procedures involve providing appropriate mixtures containing Gβγ. Such mixtures include sub-cellular mixtures or mixtures prepared based on a purified phosducin variant and components of G-protein-mediated signalling pathways.

Interaction of a compound of the invention with Gβγ can also be considered through NMR based methodology according to Shuker et al., Science 274:1531-1534 (1996). In one such embodiment, a specific compound of the invention is considered regarding its binding properties for Gβγ. The assay starts with contacting a mixture containing a specific compound of the invention such as compound 1, compound 2 or compound 3 with a $^{15}$N-labeled Gβγ. Binding of the compound to Gβγ can be determined by monitoring the $^{15}$N- or $^{1}$H-amide chemical shift changes in two dimensional $^{15}$N-heteronuclear single-quantum correlation ($^{15}$N-HSQC) spectra. A further mixture containing a further compound of the invention is then contacted with a $^{15}$N-labeled Gβγ and binding of the test compound to Gβγ can be determined as above. Since such NMR spectra can be rapidly obtained, it is feasible to consider a large number of compounds. A compound may then be identified which binds to Gβγ according to the specific compound of the invention such as compound 1, compound 2 or compound 3. A representation of this compound may be stored in the database.

The effect of the compound of the invention on Gβγ may also be assayed in a living cell that contains Gβγ. Specifically, a method may be used wherein it is considered whether the compound increases the contractility of muscle cells, said method comprising the following steps:

(a) measuring the contractility of isolated muscle cells after stimulation, preferably with a β-adrenergic receptor agonist,

(b) measuring the contractility of isolated muscle cells according to (a), whereby said muscle cells are further exposed to a compound compound of the invention, and

(c) determining the quantitative increase of the addition of a compound of the invention by comparison of contractility in step (b) and in step (a).

The compound of the invention is tested under conditions in which Gβγ signalling is activated, e.g. by providing a β-adrenergic receptor agonist (e.g. adrenaline, noradrenaline). A representation of compounds causing improved contractility in step (b) than in step (a) above may be entered into the database. Any muscle cell may be used, preferably a heart muscle cell.

Other selecting techniques include a method for identifying a compound inhibits a specific Gβγ-mediated process, comprising the following steps:

(i) incubating a mixture comprising Gβγ and a downstream component of the specific Gβγ-mediated process in predefined concentrations, whereby said component is controlled by a Gβγ mediated process in the mixture,

(ii) incubating, under conditions as in (i), a mixture comprising Gβγ, said downstream component of a Gβγ-mediated process and a compound of the invention, and

(iii) selecting a compound of the invention which inhibits Gβγ in said specific Gβγ-mediated process.

Other selecting techniques include a method for identifying a compound which inhibits specific Gαq-mediated processes, comprising the following steps:

(i) incubating a mixture comprising Gαq or any Gβγ independent GRK-2 substrate and a downstream component of a specific Gαq- or Gβγ independent GRK-2 substrate- mediated process in predefined concentrations,
(ii) incubating, under conditions as in (i), a mixture comprising Gαq or any Gβγ independent GRK-2 substrate, a downstream component of a specific Gαq- or Gβγ independent GRK-2 substrate- mediated process, and a compound of the invention, and
(iii) selecting a compound of the invention which does not inhibit a Gαq- mediated process or Gβγ-independent GRK-2 phosphorylation.

Still other selecting techniques include a method for identifying a compound which inhibits Gβγ-mediated processes in cells, comprising the following steps:

(i) incubating cells with an agonist of a $G_i$-protein-coupled receptor and measuring a signal due to the amount or activity of a component of a Gβγ-mediated process,
(ii) incubating cells, under conditions as in (i) with said agonist and a compound of the invention and measuring said signal due to the amount or activity of said component of said Gβγ-mediated process, and
(iii) selecting a compound which results in a lower amount or activity of said component in step (ii) than in step (i).

Still other selecting techniques include a method for identifying a compound of the invention which does not inhibit Gαq-mediated processes in cells, comprising the following steps:

(i) incubating cells with an agonist of a Gq-protein-coupled receptor and measuring a signal due to the amount or activity of a component of a Gq-mediated process,
(ii) incubating cells, under conditions as in (i) with said agonist and a compound of the invention and measuring said signal due to the amount or activity of said component of said Gq-mediated process, and
(iii) selecting a compound which results in an essentially unchanged amount or activity of said component in step (ii) than in step (i).

The amount (and/or activity) of a reporter produced in the absence and presence of a compound of the invention is determined and compared. A preferred reporter is inositol 1,4,5-triphosphate (IP3) which can be quantified using a commercial kit. In these techniques, compounds may be contacted with mixtures or cells, whereby a second messenger response, e.g. IP3, cAMP or $Ca^{2+}$, is then measured to determine whether the potential compound activates or inhibits Gβγ.

**Materials and methods**

**Rhodopsin phosphorylation**

All substances derived from the virtual screen were tested for their ability to inhibit the Gβγ-dependent activity of G protein-coupled receptor kinase 2 (GRK-2) in a rhodopsin phosphorylation assay. Stock solutions of 10mM of all compounds were dissolved in DMSO, for all assays (in vitro and cellular) DMSO was added in the same amount to the respective control group. The initial compound screen was carried out in a 96 well plate formate. The assay mix contained 15μM ATP, 10mM $MgCl_2$, 20nM Gβγ (from bovine brain), 60nM GRK-2(purified from baculoviral culture), $^{32}P$-ATP (approximately 200.000 cpm/assay point) and 10μM of the respective compound in 50μl assay buffer (20mM Hepes pH 7.2, 2mM EDTA); 600nM rhodopsin (purified from bovine retina) was added in the dark. Stimulation was achieved by incubation for 10-15 min under yellow light. Subsequently, the mix was aspirated with a cell harvester and applied onto a glass-fiber membrane (GF/C); then, the assay plate was washed 10 times with 10ml assay buffer each. The membrane was counted in a Wallac counter (Cerenkov). Additionally, 45μl of the assay mix were counted to determine the total amount of radioactivity in the assay. As a control, the compounds were omitted from the assay mix and only 5μl of 1% DMSO were added to achieve an equal concentration of 0.1 % DMSO in all wells. Total inhibition with 30μM heparin was used as positive control. Less than 75% of control GRK-2 activity was considered as a hit.
Positive candidates were tested in a similar assay, where the reaction mix was applied to a gel, which was subsequently exposed to an X-ray film for autoradiography. Different concentrations of all compounds were tested for their influence on GRK-2 activity. Concentrations of the assay components were: GRK-2 20-50nM, Gβγ 3-1000 nM, rhodopsin 100-400nM.

**Tubulin phosphorylation**

Phosphorylation of tubulin β3 (400nM or, in the case of COM1, 3μM) by 50nM of GRK-2 was performed in the absence of GAII compounds were diluted in assay buffer (20 mM Hepes, pH 7.2; 2 mM EDTA), and the phosphorylation reaction was started by adding a mix of GRK-2, tubulin β3 from porcine brain, $MgCl_2$ and ($^{32}P$)-ATP to the compound of interest to yield the indicated final concentrations. Then, the assay mix was incubated for 30 min at 30°C before aplying it onto a gel which was subsequently exposed to an X-ray film for autoradiography.

**Cells and media**

A chinese hamster ovary (CHO)-FRT cell line stably expressing the mouse alpha-2 A receptor was created using the Invitrogen Flpln™ system. The cDNA for the mouse alpha 2A receptor with a flag-tag at its C-terminus (amino acid sequence: DYKDDDDK) was cloned into the pcDNA™ 5 /FRT vector (Invitrogen) using the Kpnl/BamHl restriction sites of the multiple cloning site. The CHO Flp-In™ host cell line has a single Flp Recombinase Target (FRT) site located at a transcriptionally active genomic locus and was purchased from Invitrogen. An isogenic expression cell line was generated by co-transfecting the Flp-In™ host cell line with the Flpln expression vector and the Flp recombinase expression vector, pOG44 (Invitrogen). The transiently expressed recombinase integrated the expression vector at the genomic FRT site. Cells expressing the 2A receptor were selected with hygromycin B (Merck), since the hygromycin B resistance gene had been inserted into the cellular genome together with the transgene. To ensure the homogeneity of the cell population, the cells were additionally subcloned. Single clones were raised and tested for expression of the alpha2A receptor by western blotting with an anti-flag antibody-peroxidase conjugate (Sigma) using standard protocols. The CHO cells were grown in Hams-F12 medium (Biochrom) supplemented with 10% fetal calf serum (Invitrogen), penicillin 100U/ml, streptomycin 100μg/ml (Invitrogen), L-glutamine 2mM (Invitrogen) and hygromycin B (Sigma) 500μg/ml. HEK 293 (ATCC CRL-1573) cells were grown in DMEM (Invitrogen), supplemented with 10% fetal calf serum (Invitrogen), penicillin 100U/ml, streptomycin 100μg/ml (Invitrogen) and L-glutamine 2mM (Invitrogen). All cells were incubated in a humidified atmosphere at 5% $CO_2$ and 37°C.

**Isolation of single adult cardiomyocytes from rabbit hearts**

Female New Zealand White rabbits (12 weeks old) were anesthesized with thiopental (75-1 00mg/kg) and 500 U heparin were injected intravenously. The thorax was opened and the heart was excised and put into ice-cold NaCl solution (0.9%). Fat and pericardium were removed. The aorta was attached to a perfusion system connected to a pump and the heart was retrogradely pefused at a flow rate of 20-40 ml/min with oxygenated (95% oxygen, 5% $CO_2$) Powell medium (110mM NaCl, 2.5mM KCl, $KH_2PO_4$ 1.2 mM, $MgSO_4$ 1.2 mM, $NaHCO_3$ 25 mM, Glucose 11 mM; pH 7.4) until all blood was washed out. The solution was exchanged to the same buffer containing 0.5-1 mg/ml collagenase type II (Worthington) and 40μM $CaCl_2$; then, the perfusion was continued for additional 5-15 minutes. The digested myocardium was cut into small pieces and suspended in Powell medium, single myocytes were obtained by bubbling carbogen gas (95% oxygen, 5% carbogen) through the solution. The cell suspension was filtered through a 200μm nylon mesh and cells were sedimented. The cell pellet was resuspended in Powell medium containing 0.2 mM calcium. Then, cells were again sedimented and resuspended in Powell medium with 0.4 mM calcium. This cell suspension was layered over Powell medium with 40mg/ml BSA and 1mM calcium, and the myocytes were sedimented and the pellet resuspended in M199 culture medium (M199 supplemented with 1x MEM vitamines, 1x MEM non-essential amino acids, penicillin 100U/ml streptomycin, 100μg/ml, gentamycin 100μg/ml, insulin 10μg/ml, Hepes 25mM) and plated on laminin-coated dishes (2-5 μg/$cm^2$) at a density of $1x10^5$cells/$cm^2$. Measurements of cell contractilty and cAMP formation were performed 24h after plating.

**Cytotoxicity assay**

Cells in buffer or medium with or without compounds were incubated for 4h at 37°C with 1/10 volume of WST-1 reagent (Roche). After mixing for 1 min, the absorbance at 440 nm was determined in a Tecan plate reader. As blank controls, only medium or buffer incubated with WST-1 was used. Absorbance values from cells incubated without compounds were regarded as 100% viability and values below 75% of the control were considered as toxic compounds.

Calcium measurements

Cells were grown in monolayers for at least 24h. Then, the cells were detached using trypsin/EDTA (Biochrom) and the cell pellet was washed two times with Krebs-Hepes buffer (NaCl 118 mM, KCl 4.7 mM, $MgSO_4$ 1.2 mM, $KH_2PO_4$ 1.2 mM, $NaHCO_3$ 4.2mM, D-glucose 11.7 mM, $CaCl_2$ 1.3 mM, Hepes 10 mM, pH 7,4). Subsequently, the cells were

loaded with 5µM Fura-2 AM (Molecular Probes) in Krebs-Hepes buffer containing 0.02%(w/v) Pluronic-F127 in suspension under constant shaking for 30 minutes. To remove non-resorbed FURA-2, the cells were washed three times with Krebs Hepes buffer containing 0.5% (w/v) bovine serum albumin (BSA) and finally suspended in the same buffer at a cell concentration of $1x10^6$ cells /ml. 100µl of this cell suspension were plated in each well of a black 96 well plate (NUNC) and 90 µl of the desired compound in Krebs-Hepes buffer + BSA were added. The cells were incubated for at least 30 minutes at room temperature before the measurement. The calcium response following receptor stimulation by the respective agonist was monitored by using a BMG Polar Star™ plate reader. Using dual wavelength excitation at 340nm and 380nm and measuring the emission at 510nm for each excitation wavelength, the receptor was stimulated by adding 10µl agonist solution (for alpha-2A receptors: UK14304 (10nM-1µM); for muscarinic receptors: carbachol (1-100µM), both from Sigma) in Krebs Hepes buffer + BSA via the system pumps of the instrument. The ratio of the emission at 510 nm of the two excitation wavelengths (340nm/380nm) reflects the intracellular calcium concentration. As a control for each intervention group, a control curve was created by only injecting buffer and this curve was substracted from the agonist curve. The maximum increase in $Ca^{2+}$ was calculated as the maximum of this blank-corrected curve minus the basal point of the curve before injection.

**IP3 measurements**

Inositol phosphates in whole cells were determined by using assay kits from Perkin Elmer (cat. no. 6760621), in combination with GST detection kits (cat. no. 6760603x) designed for the ALPHA screen™ technology (amplified luminescent proximity homogeneous assay). The assays were performed according to the manufacturer's instructions.

In detail, CHO-alpha2AR cells were grown for at least 24 h and detached using Versene (Invitrogen), washed once with PBS/Hepes buffer (PBS with 15mM Hepes, pH 7.4) and resuspended in PBS/Hepes. The cell number was adjusted to $6x10^5$ cells/ml with PBS/Hepes and the cells were incubated with the compound at the desired concentrations for 30 min at 37°C under gentle agitation. Subsequently, 3000 cells were plated in a white round-bottom 96 well assay plate in 10µl and 10µl of the respective agonist was added in PBS/Hepes. The reaction was stopped after 30 sec by adding 10µl 1.05% perchloric acid. After an incubation of 30min, 60µl/well of IP3 binding protein (0.033U/µl; Perkin Elmer) in Tris buffer (100mM Tris, pH 8.7, 0.1% Tween-20, 0.1 % BSA) were added and the assay mix was incubated for 30 min in the dark at room temperature. A pre-incubated alpha screen™ bead mix for IP3 assays (Perkin Elmer) was added at 10µl/well (10 nM biotinylated IP3 analog, 10 µg/ml streptavidin donor beads, 10 µg/ml anti-GST acceptor beads in Tris buffer; all from Perkin Elmer). After 1 hour incubation at room temperature in the dark, the plate was analysed in a Fusion alpha™ plate reader (Perkin Elmer)

**cAMP assays**

Similarly, cyclic AMP formation in whole cells was determined by using assay kits from Perkin Elmer (cat. no. 6760600). The assays were performed according to the manufacturer's instructions.

Calcium tolerant single adult rabbit cardiomyocytes were plated 24h prior to the experiment in 96 well plates coated with laminin. The culture medium was removed carefully and a mix of the respective compounds (10-30µM), buffer control or isoproterenol at concentrations ranging from $1 x10^{-10}$ M to $1 x10^{-6}$ M and anti-cAMP-acceptor beads (15 µg/ml, Perkin Elmer) was pipetted onto the cells in 40µl stimulation buffer HBSS (containing 0.5 mM IBMX, 5mM Hepes, 0.1% BSA, pH 7.4). The stimulation period was 30 min at 37°C. Subsequently, a pre-incubated assay mix containing streptavidin donor beads (20 µg/ml) and biotinylated cAMP (10 nM, all from Perkin Elmer) in 60 µl lysis buffer (5 mM Hepes, pH 7.4, 0.1% BSA, 0.3% Tween-20) was added and the assay plate was incubated in the dark at room temperature for 1 hour. Before analysis in a Fusion alpha™ plate reader (Perkin Elmer) using the ALPHA screen™ program, 25µl of each well from the 96 well plate were transferred to one well of a white 384 well plate (Corning).

**Measurement of cell shortening**

Cell shortening was measured with an electro-optical monitoring system connected to an online digitalized assessment of amplitude and velocity of shortening and relaxation (Scientific Instruments). The myocytes cultured on laminin-coated plastic cover slips were transferred to a single cell investigation system (Scientific Instruments) in a temperature-controlled cuvette (37°C), at a constant medium flow of 0.5 ml/min with a calcium containing tyrode (NaCl 126mM, KCl 5.4mM, $MgCl_2$ 1mM, $CaCl_2$ 1.8mM $NaH_2PO_4$ 0.33mM, Glucose 10mM, Hepes 10mM, pH 7.4) and under constant electrical stimulation (50 V and an 800 msec pulse to achieve a contraction frequency of 70/min). After the contraction amplitude reached stability, the experiment was started by perfusing tyrode containing the respective compound in the desired concentration for 10min and subsequently stimulating the cells with increasing concentrations of the -receptor agonist isoproterenol ($1x10^{-9}$M to $1 x10^{-7}$ M) in tyrode+compound. The control group was subjected to the same protocol in the absence of any compound. Cell shortening was monitored using an edge detection camera

and the data were transferred to a computer and fractional shortening was calculated for a single cell.

**Examples**

To test the ability of the compounds to inhibit Gβγ-dependent signalling a rhodopsin phosphorylation assay may be used. Upon stimulation, rhodopsin (the anolog of the β-adrenergic receptor in the retina) is phosphorylated by GRK-2. This process depends on the presence of Gβγ. The compound concentration in this screen was 10 μM, and compounds showing an at least 30% inhibition of GRK-2 activity are especially preferred. The effect of different compound concentrations may be determined in the same assay. GRK-2-mediated phosphorylation of rhodopsin is inhibited by tested compounds in concentrations ranging between 1 and 10 μM (Figure 1). To test the specificity of this inhibition and to exclude unspecific effects of the compounds on assay components, the inhibitory effects of the compounds may be counterchecked by increasing substrate concentrations at fixed compound concentrations. Figure 4 shows that the inhibition of Gβγ-dependent GRK-2 activity could indeed be reversed by increasing substrate concentrations.

It may be shown that the observed inhibition of GRK-2 activity is truely Gβγ-dependent by considering the effect of the compounds on the ability of GRK-2 to phosphorylate the Gβγ-independent GRK-2 substrate tubulin-β3. Figure 2 shows that tubulin-β3 phosphorylation was not inhibited by the compounds when applied at concentrations sufficient to inhibit the Gβγ-stimulated rhodopsin phosphorylation.

As a second method to demonstrate the Gβγ dependency of the observed inhibition, rhodopsin phosphorylation assays may be repeated in the presence and absence of the compounds at increasing concentrations of Gβγ (3 -1000nM). Figure 3 shows that the inhibitory effect of COM2 on GRK-2 activity was indeed reversed with increasing concentrations of Gβγ, and completely blunted at a concentration of 1000nM of Gβγ.

The *in vivo* effects of the compounds of the invention can be measured in different cellular assays. Lack of cytotoxicity may be shown using a WST-1 assay, a standard assay of cell viability. A tetrazolium salt (WST-1) is cleaved by cellular enzymes to a formazan-dye, which can be detected photometrically. The amount of dye formed is proportional to the number of metabolically active cells. The effect of the compounds on the cells was determined under the same assay conditions as used in the respective assays for each cell type (lower panels of Figures 5 and 9).

Phospholipase C-β2 (PLC-β2) is another effector of Gβγ. GPCRs (G-protein coupled receptors) which are coupled to inhibitory G-proteins ($G_i$) mediate their signalling by initiating the dissociation of Gβγ from Gαi. The released Gβγ subunits subsequently stimulate PLC activity. Therefore, compounds which affect Gβγ should also have an influence on the amount of inositol phosphates formed by PLC-β2. A CHO cell line was created which overexpresses the mouse α-adrenergic receptor type 2A, a receptor only coupled to $G_i$. First, the influence of the compounds on inositol phosphate levels is determined in this cell line: The cells are stimulated for 30 sec with 10μM UK14304, a selective alpha2 receptor agonist, and the formation of inositol phosphates was monitored using the ALPHA screen™ assay technology. Figure 5 shows that the addition of the compounds actually resulted in decreased accumulation of inositol phosphates.

Downstream of the second messenger molecule IP3, intracellular calcium levels are regulated by receptor stimulation. Therefore, the effects of the compounds were additionally tested on intracellular calcium release following receptor stimulation. Figure 7 shows an example of the effects of two compounds on calcium transients in stimulated CHO alpha2A receptor-expressing cells. Intracellular calcium release is decreased after pre-incubation with 30μM of the respective compound for 30 minutes. These results corroborate the findings that inositol phosphate formation is decreased by the same compounds. In order to test the Gβγ specificity of this inhibition, additional cellular assays were performed. The influence of the compounds on calcium release mediated by a Gβγ-independent signalling pathway was determined in 293 cells by stimulating the native muscarinic M1 receptor with carbachol and measuring calcium transients. The M1 receptor is coupled to a Gq-protein which activates PLC by its alpha subunit, so that intracellular calcium signalling is induced independently of Gβγ. Figure 8 shows that COM2 does not have an effect on the calcium release mediated via the $G_q$-coupled pathway as it had on the $G_i$-mediated signal, whereas COM3 displays an inhibitory potential also in this assay indicating a broader, less specific mode of action of this compound.

Another parameter is the influence of the substances on intracellular cAMP formation, another important second messenger system in cardiomyocytes. As the compounds are derived from an in silico modelling which mimicks the interaction of Gβγ with its physiological binder phosducin, a similar effect on cAMP formation as described for phosducin overexpression may be expected. Phosducin is known to have no or even a small inhibitory effect on cAMP signalling and the same was observed for the compounds. The measurement of basal and isoproterenol-stimulated cAMP levels in isolated adult rabbit cardiomyocytes did not show any significant effect of any compound compared to the control group (figure 9).

Overexpression of proteins which can bind Gβγ subunits, such as phosducin or the C-terminus of GRK-2, result in an increased contractility of cardiomyocytes. Therefore, the compounds' effects on this important parameter were determined. In isolated rabbit cardiomyocytes, basal, compound-incubated and isoproterenol- stimulated contractility were measured and expressed as fractional shortening of the single cell. Figure 10 shows the positive inotropic

effect of COM1 on isoproterenol-stimulated contractility of the cells.

In summary, the compounds of the invention show an inhibition of Gβγ-dependent effects both in cell-free and in cellular assays. Additionally, the compounds increase the contractile reserve of ventricular cardiomyocytes without stimulating cAMP levels.

## Claims

**1.** A compound of of the following formula (I) or a pharmaceutically acceptable salt or ester thereof for use as a medicine:

A-L-B (I)

wherein A and B, which may be the same or different are represented by the following group (II)

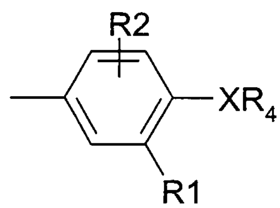

(II)

wherein

X represents -CONR3-, -NR3CO-, or -NR3COCH=CH-;

L represents

a single bond,
an oxygen atom,
a sulfur atom,
a $CR^5R^6$ group or a $NR^5$ group wherein $R^5$ and $R^6$ are selected from a hydrogen atom, an alkyl group and a halogen atom,

R1 and R2 which may be the same or different represent

a hydrogen atom,
a carboxyl group,
a carboxamide group,
a group COOR7, wherein R7 represents

an alkyl group,
an aryl group,
an aralkyl group,

R3 represents

a hydrogen atom,
a $C_{1-7}$ acyl group,
a $C_{1-4}$ alkyl group which may be substituted by a halogen atom;

R4 represents

an aromatic carbocyclic or heterocyclic ring, which may be substituted by one or more substituents selected from the group of

halogen atoms,
alkyl groups,
cycloalkyl groups,
hydroxyl group,
alkoxy groups,
cycloalkoxy groups,
amino group,
alkylamino group,
dialkylamino group,
cycloalkylamino group,
carboxyl group,
alkoxy carbonyl groups,
alkylamino carbonyl groups,
dialkylamino carbonyl groups,
acyl groups, or

R3 and R4 represent together an alkylene group or the following fragment (III) forming together with the atoms to which it is bound a cyclic imido structure:

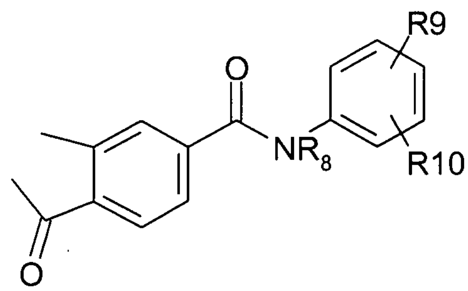

(III)

wherein

R8 represents

a hydrogen atom, or
a $C_{1-4}$ alkyl group which may be substituted by one or more halogen atoms, or an acyl group;

R9 and R10 which may be the same or different are selected from the group of halogen atoms,

alkyl groups,
cycloalkyl groups,
alkenyl groups,
alkinyl groups,
aryl goups,
aralkyl groups,
hydroxyl group,
alkoxy groups,
amino group,

aminoalkyl group,
dialkylamino group,
carboxyl group,
carboxamido group,
alkoxy carbonyl groups,
alkylamino carbonyl groups,
dialkylamino carbonyl groups,
acyl groups.

2. The compound according to claim 1 wherein A and B are the same groups of formula (II).

3. The compound of claim 1 or 2 wherein A and B are represented by the following formula (IV)

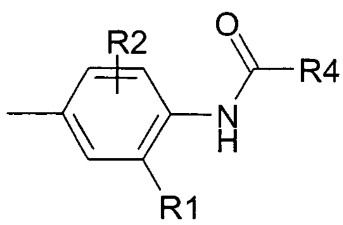

(IV)

wherein R1, R2 and R4 are as defined above.

4. The compound of any one of the preceding claims wherein the compound of formula (I) is represented by the following formula (V)

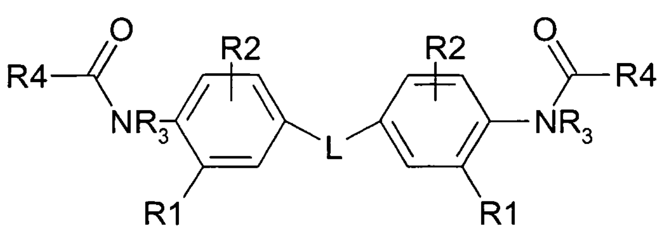

wherein L, R1, R2, R3, and R4 are a s defined above.

5. The compound of any one of the preceding claims wherein the compound of formula (I) is represented by the following formula (V)

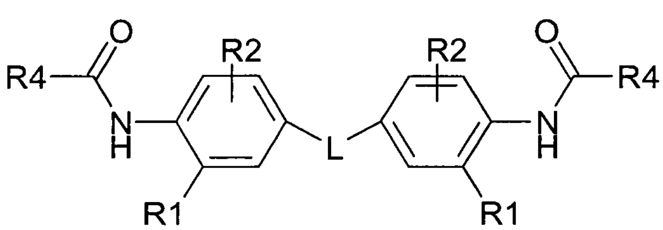

wherein L, R1, R2, and R4 are a s defined above.

6. The compound of any one of the preceding claims wherein the compound of formula (I) is represented by the

following formula (VI)

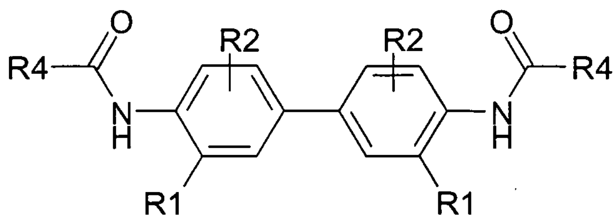

wherein R1, R2 and R4 are as defined above.

7. The compound according to any one of the preceding claims, which is

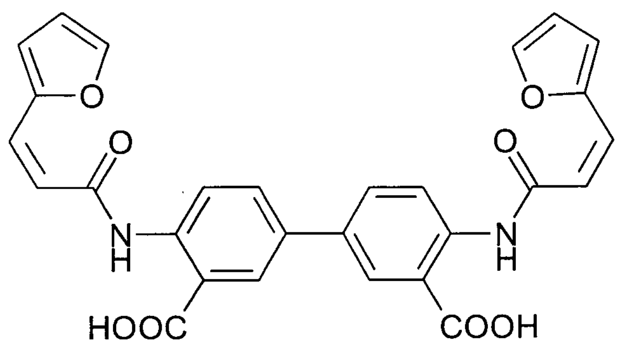

or

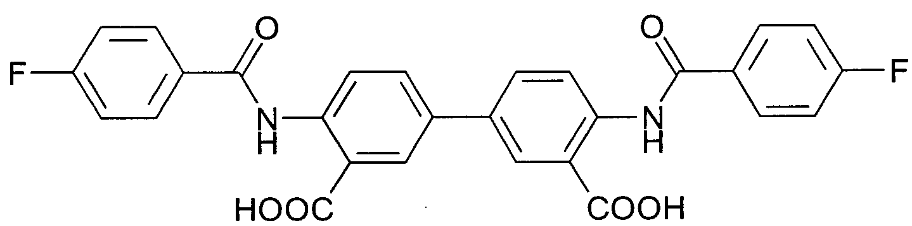

or a salt thereof.

8. The compound according to claim 1 wherein L is an oxygen atom or a $CF_2$ group.

9. The compound of claim 9, wherein R3 and R4 represent following fragment (III) forming together with the atoms to which it is bound a cyclic imido structure:

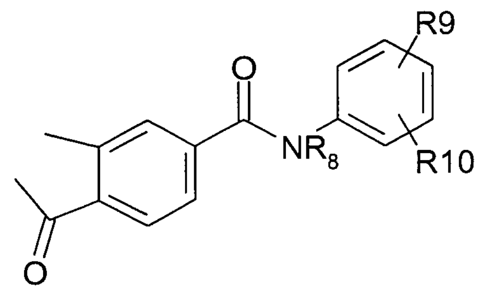

wherein R8, R9 and R10 are as defined above.

**10.** The compound of claim 9, which is

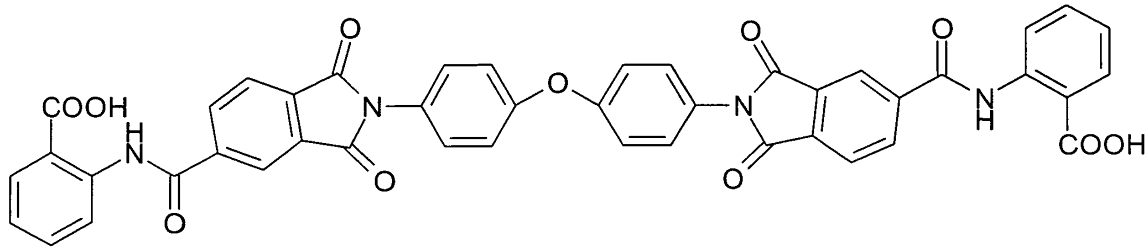

or a salt therof.

**11.** Pharmaceutical composition comprising a compound as defined by any one of claims 1 to 10 and a pharmaceutically acceptable carrier.

**12.** Use of a compound as defined in any one of claims 1 to 10 for the preparation of a medicine for the treatment or prevention of cardiovascular disease.

**13.** The use according to claim 12 wherein the cardiovascular disease is congestive heart failure.

**14.** The use according to any one of claims 12 or 13 wherein the cardiovascular disease is hypertension, myocardial ischemia, cardiomyopathy or congenital heart disease.

**15.** The use according to any one of claims 12 to 14 wherein the treatment or prevention of cardiovascular disease involves an increase of the contractility of muscle cells of the heart.

**16.** A method of producing a computer readable database comprising a representation of a compound as defined by any one of claims 1 to 10 capable of binding amino acids of a binding pocket of a Gβγ protein by noncovalent bonds, said method comprising

(a) introducing into a computer program a computer readable database comprising the threedimensional molecular structural coordinates of a binding pocket of a Gβγ protein for a compound as defined in claims 1 to 10, whereby the database is obtainable by

(a1) obtaining threedimensional structural coordinates defining said protein or the binding pocket of said protein, from a crystal of said protein or co-crystal of said protein in a complex with phosducin, and

(a-2) introducing said structural coordinates into a computer to produce a database containing the molecular structural coordinates of said protein or said binding pocket;

(b) generating a threedimensional representation of a binding pocket of said Gβγ protein in said computer program;

(c) superimposing at least one compound as defined by claim 1 to 10 on said representation of the binding pocket;
(d) assessing the binding of the compound in the binding pocket;
(e) storing a representation of the compound into a computer readable database.

**17.** The method of claim 16, wherein said binding pocket comprises βR42 to βT47, βR304 to βG310, and βW339, preferably βR42, βQ44, βM45, βR46, βT47, βV307, βL308, βG310, βW339 or βR42, βQ44, βM45, βR304, βV307, βA309, βW339.

**18.** The method of claim 17, wherein said representation is selected from the group of the compound's name, a chemical or molecular formula of the compound, a chemical structure of the compound, an identifier for the compoundand threedimensional structural coordinates of the compound.

**19.** The method of claim 18, wherein the compound of any one of claim 1 is selected by modifiying a compound as claimed in claims 7 or 10.

**20.** The method of claim 16, wherein said assessing of the compound comprises fitting the compound to said representation and performing energy minimisation.

**21.** The method of any one of claims 16 to 20, which further comprises

(f) preparing a compound represented in said computer readable database;
(g) contacting said compound in a binding assay with a Gβγ protein and
(h) determining whetherthe compound binds to the protein in the assay; and
(i) introducing a representation of the compound that has predetermined binding properties in said assay into a computer readable database.

**22.** A computer readable database produced according to any one of claims 16 or 21.

**23.** A machine readable medium embedded with the database of claim 23.

**24.** A compound according to any one of claims 1 to 10 whose representation is contained in the computer readable database of claim 22.

**25.** A compound according to any one of claims 1 to 10 or 24, **characterised by** inhibiting the Gβγ-dependent activity of G protein-coupled receptor kinase (GRK-2) by more than 25 % in a rhodosin phosphorylation assay comprising the following steps:

(a) adding rhodopsin to a mixture containing Gβγ , GRK-2, ATP, and the compound;
(b) measuring the phosphorylation activity; and
(c) comparing the phosphorylation activity measured in step (b) with the phosphorylation activity of a mixture according to step (a) which does not contain the compound.

**26.** A compound according to any one of claims 1 to 10, 24 or 25, **characterised by** increasing the contractility of a heart muscle cell *in vitro* and/or *in vivo.*

**27.** A compound according to any one of claims 1 to 10, or 24 to 26, **characterised by** increasing the intracellular calcium concentration of a heart muscle cell.

**28.** A compound according to any one of claims 1 to 10, or 24 to 27, **characterised by** decreasing IP3 of a heart muscle cell.

**29.** A compound according to any one of claims 1 to 10, or 24 to 28, **characterised by** not having an infuence on cAMP in a heart muscle cell.

Inhibition of rhodopsin phosphorylation by COM1-3

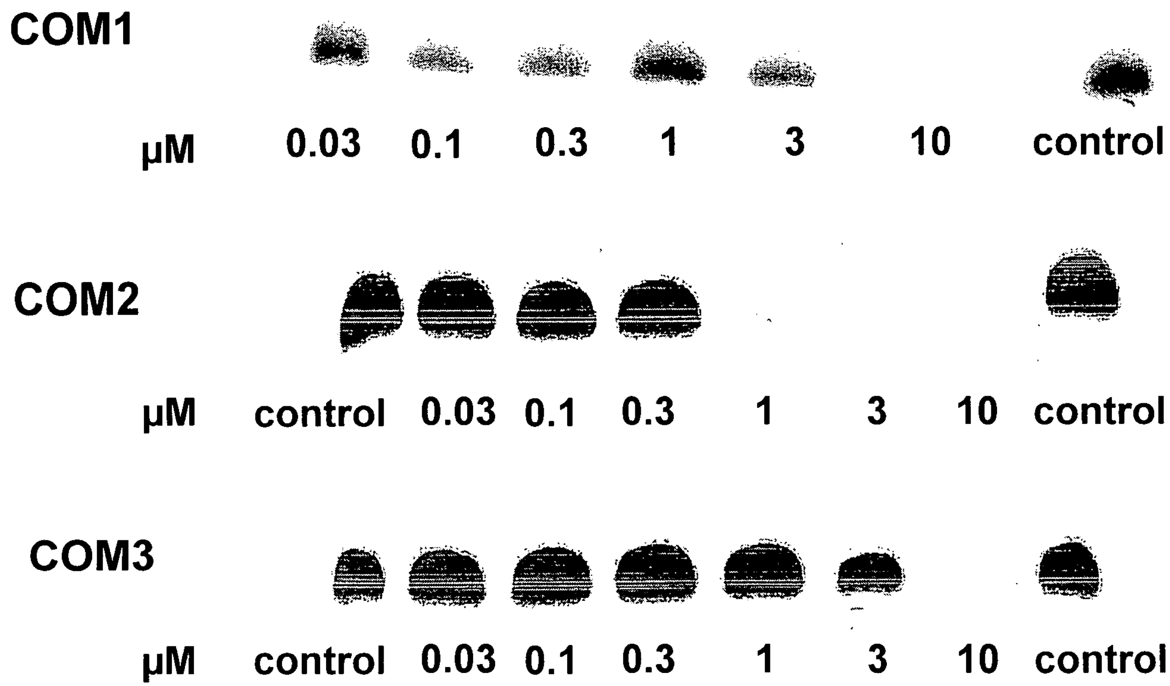


Fig. 1

Inhibition of tubulin phosphorylation by COM1-3

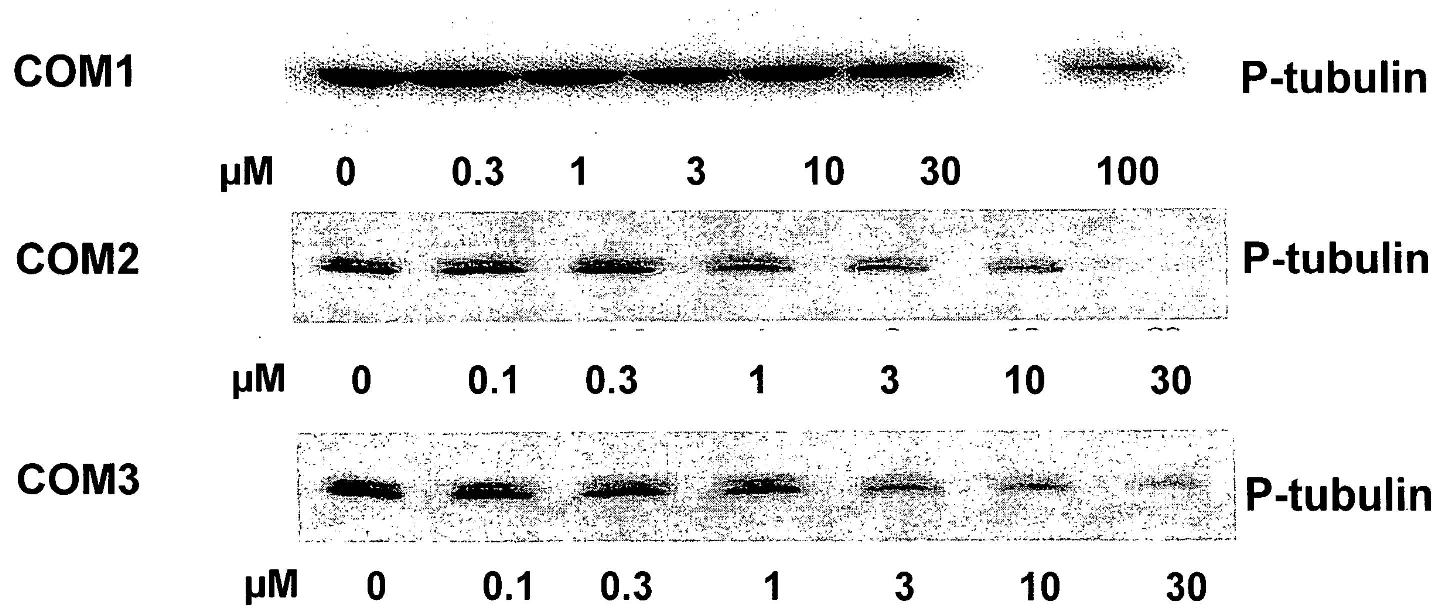


Fig. 2

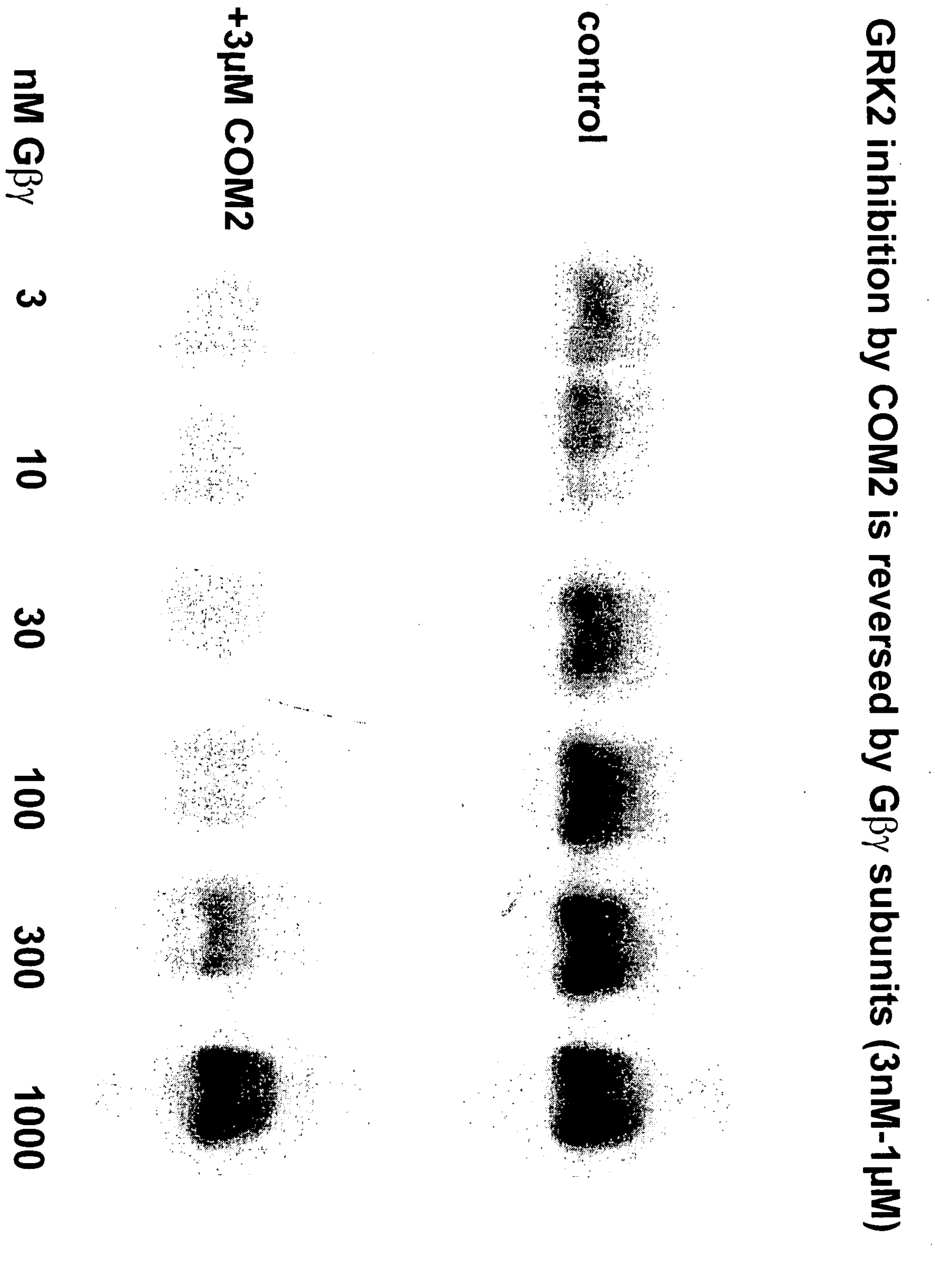
GRK2 inhibition by COM2 is reversed by Gβγ subunits (3nM-1μM)
control
+3μM COM2
nM Gβγ
3
10
30
100
300
1000

Fig. 3

**GRK2 inhibition by COM1 is reversed by its substrate rhodopsin**

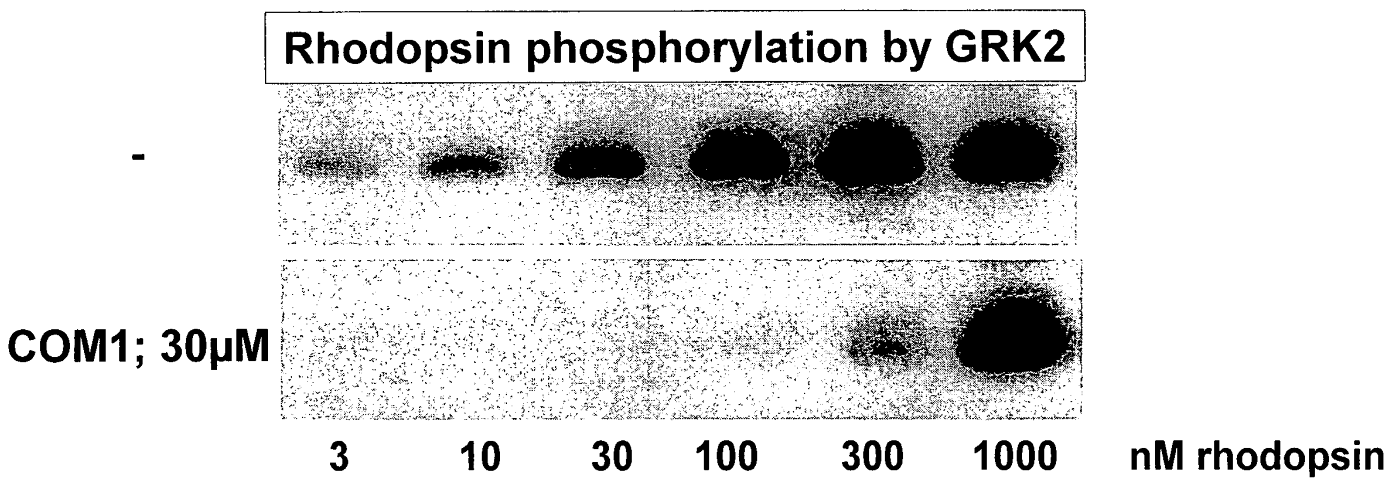


Fig. 4

Effect of COM2-3 on inositol phosphate levels in CHO alpha2A receptor cells

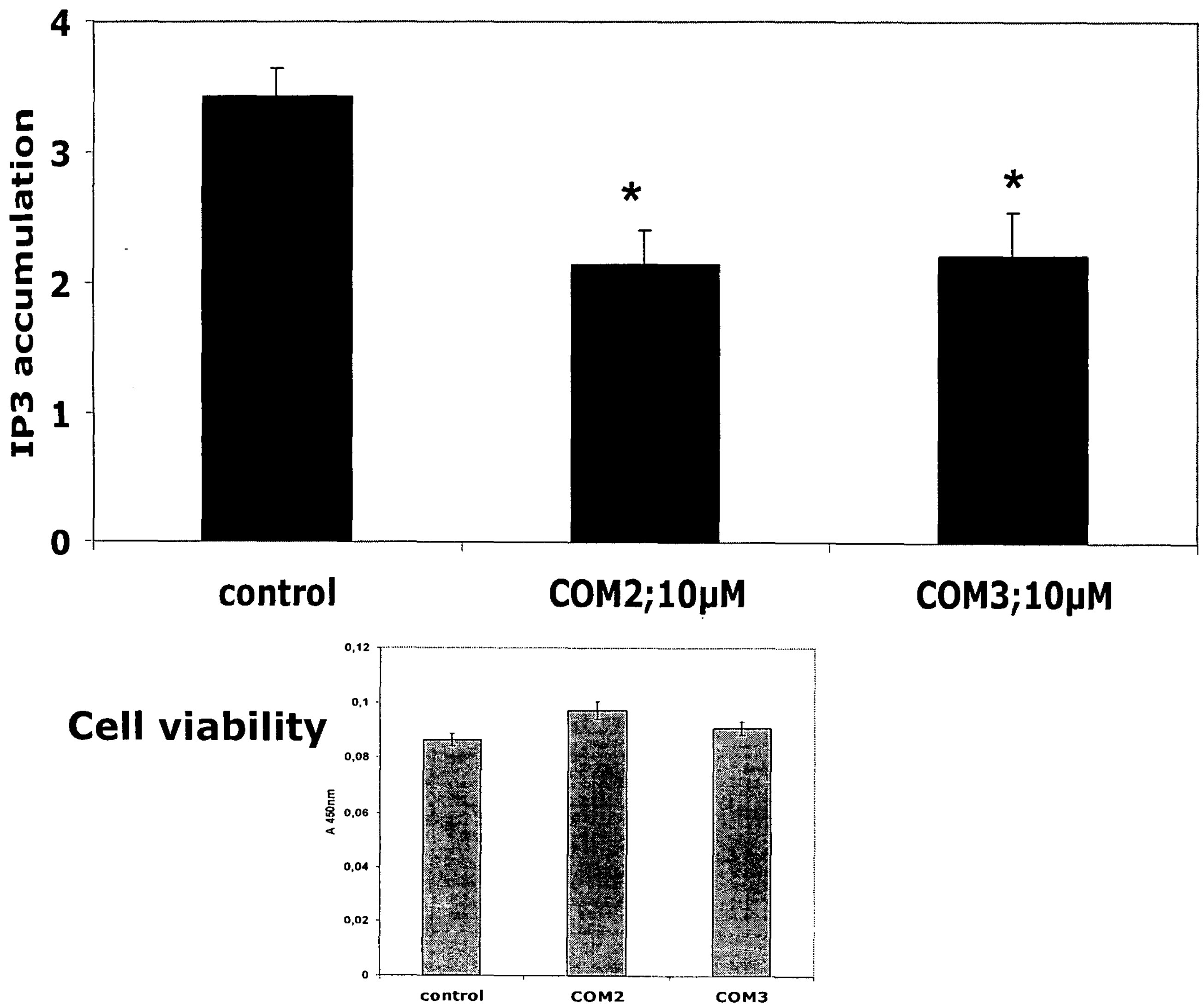


Fig. 5

## Specificity of the calcium signal in CHO α2AR cells after stimulation with UK14304

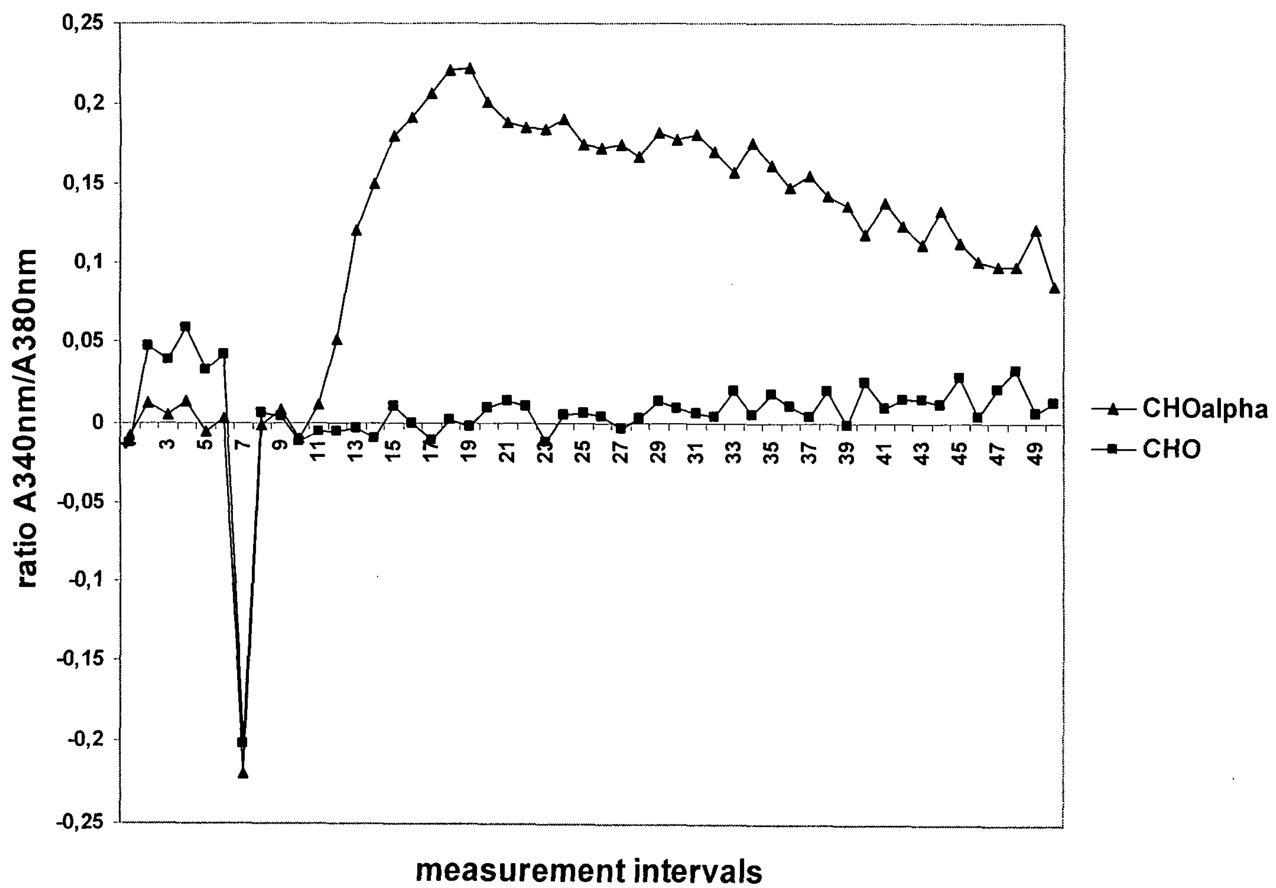


Fig. 6

**Effect of COM1-3 on calcium formation after stimulation of the alpha 2A receptor; 1µM UK14304**

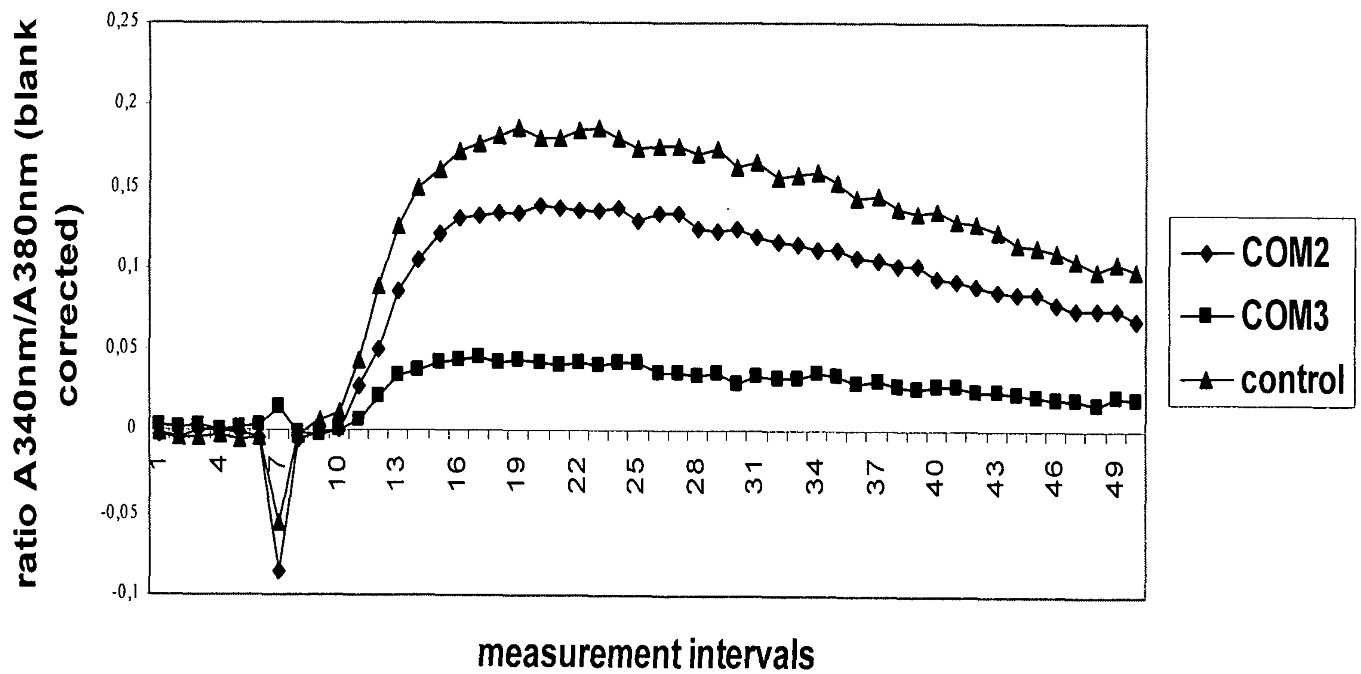


Fig. 7a

Effect of COM2 on calcium formation after stimulation of the alpha 2A receptor; 1µM UK14304

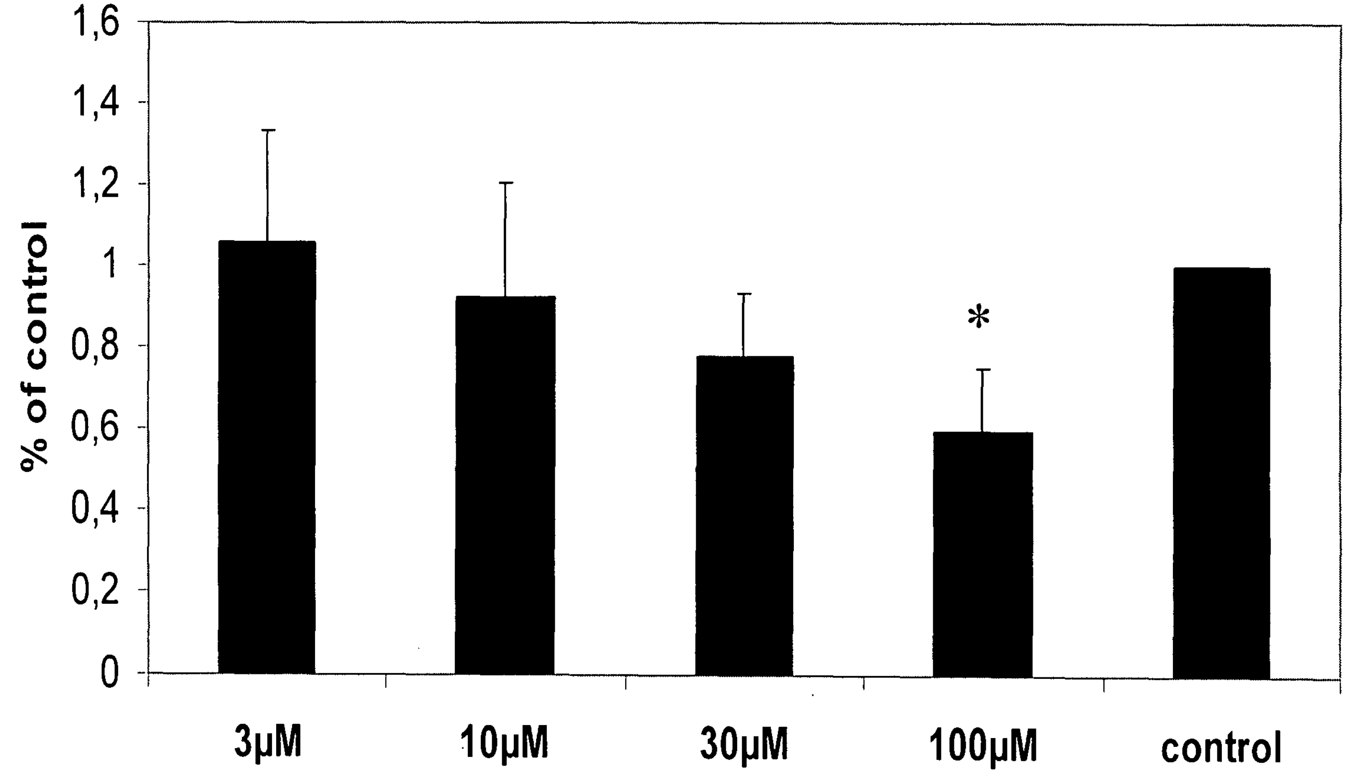


Fig. 7b

**Effect of COM2-3 on calcium formation after stimulation of the M1 receptor; 100µM carbachol**

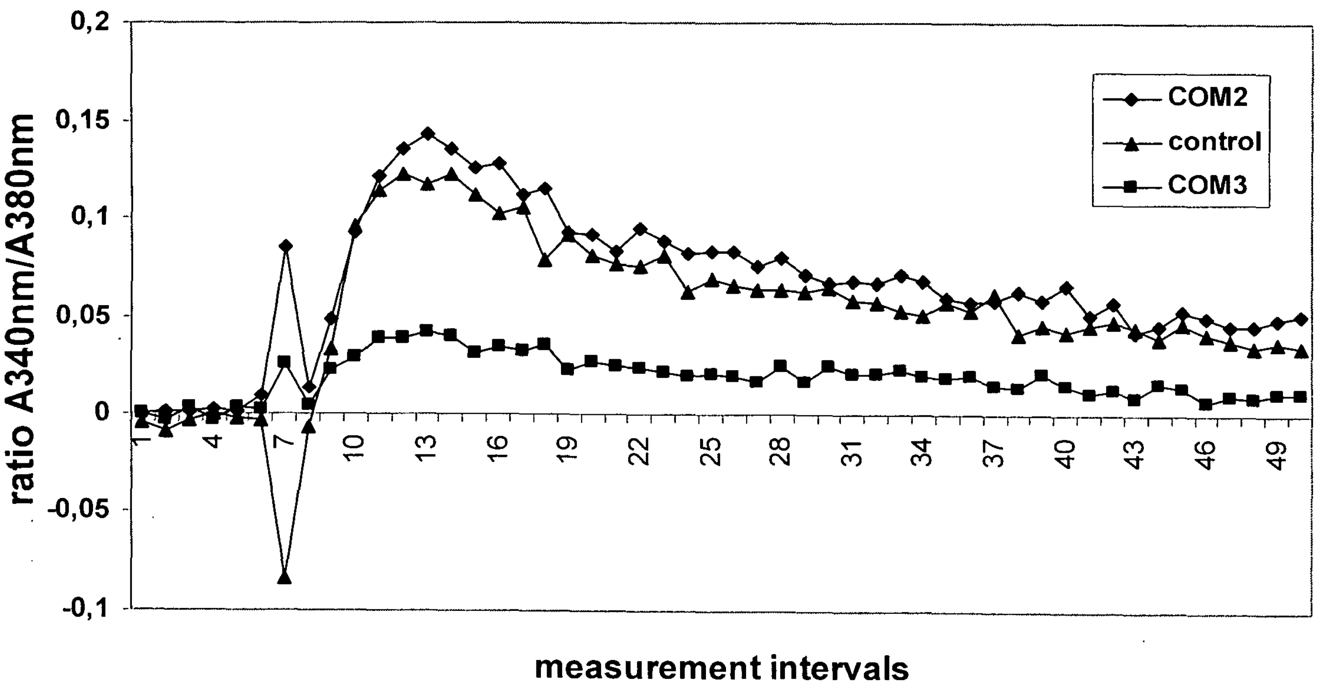


Fig. 8a

**Effect of COM2 on calcium formation after stimulation of the M1 receptor; 100µM carbachol**

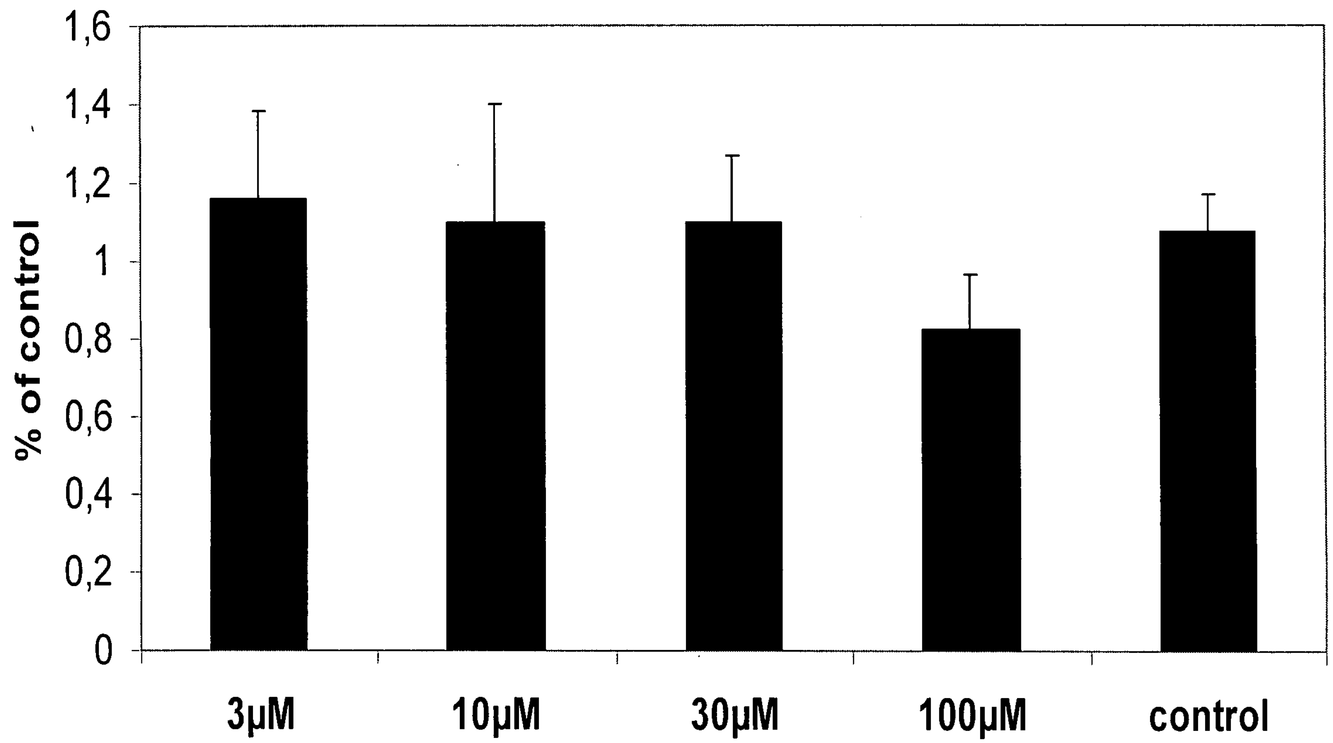


Fig. 8b

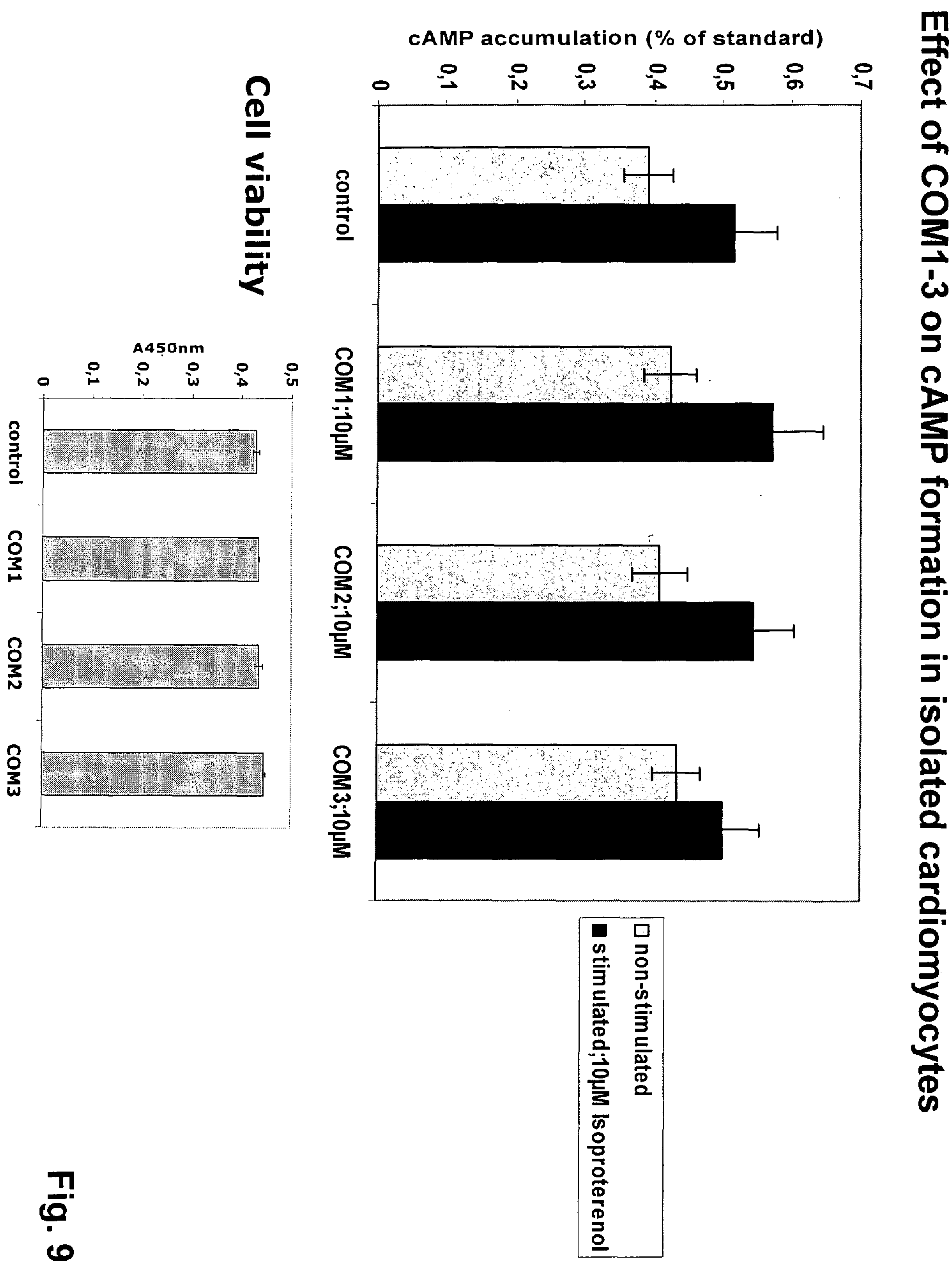
Effect of COM1-3 on cAMP formation in isolated cardiomyocytes
cAMP accumulation (% of standard)
0
0,1
0,2
0,3
0,4
0,5
0,6
0,7
control
COM1;10µM
COM2;10µM
COM3;10µM
non-stimulated
stimulated;10µM Isoproterenol
Cell viability
A450nm
0
0,1
0,2
0,3
0,4
0,5
control
COM1
COM2
COM3

Fig. 9

**Effect of COM1 on the contraction amplitude of isolated single rabbit cardiomyocytes**

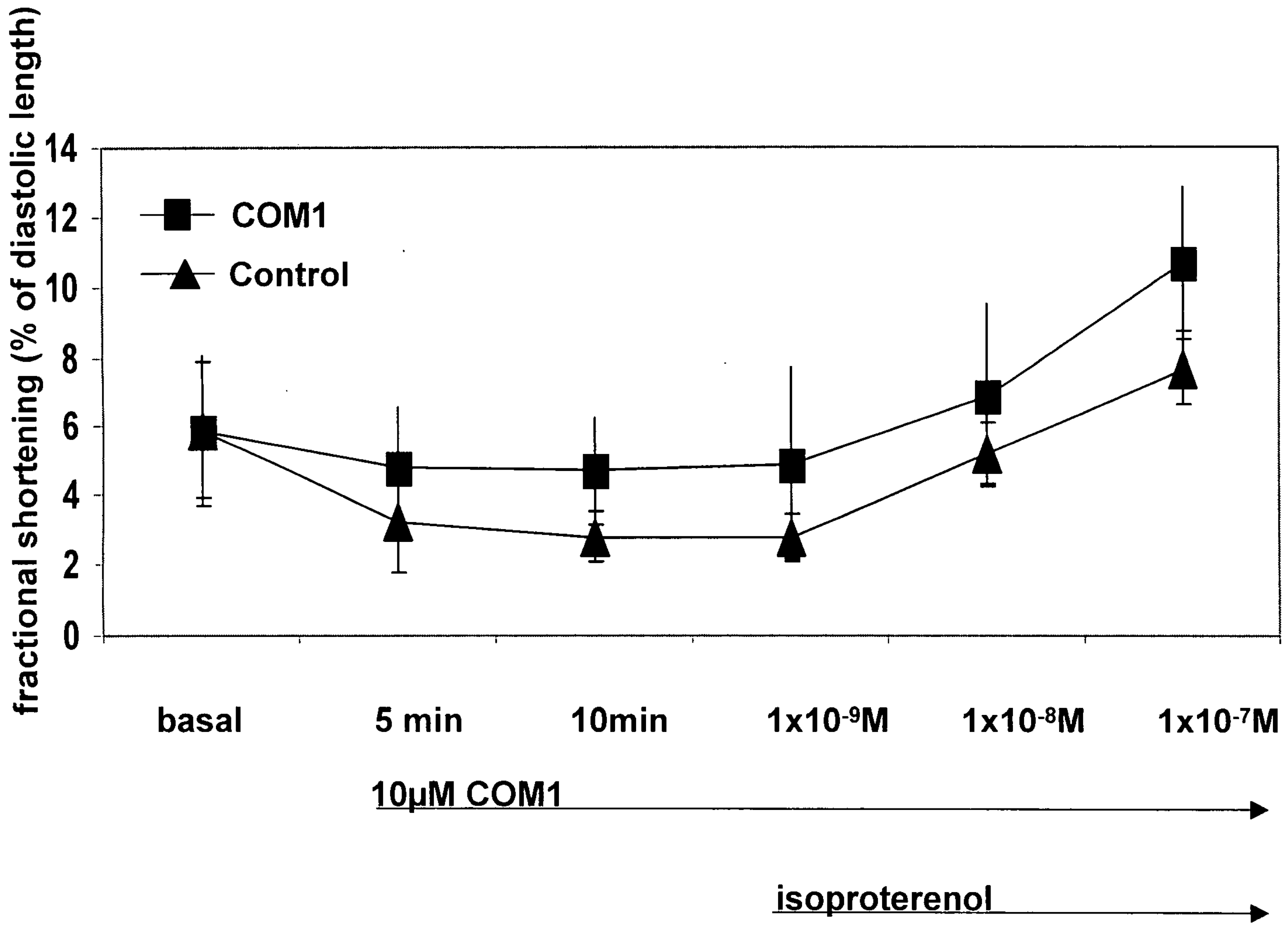


Fig. 10

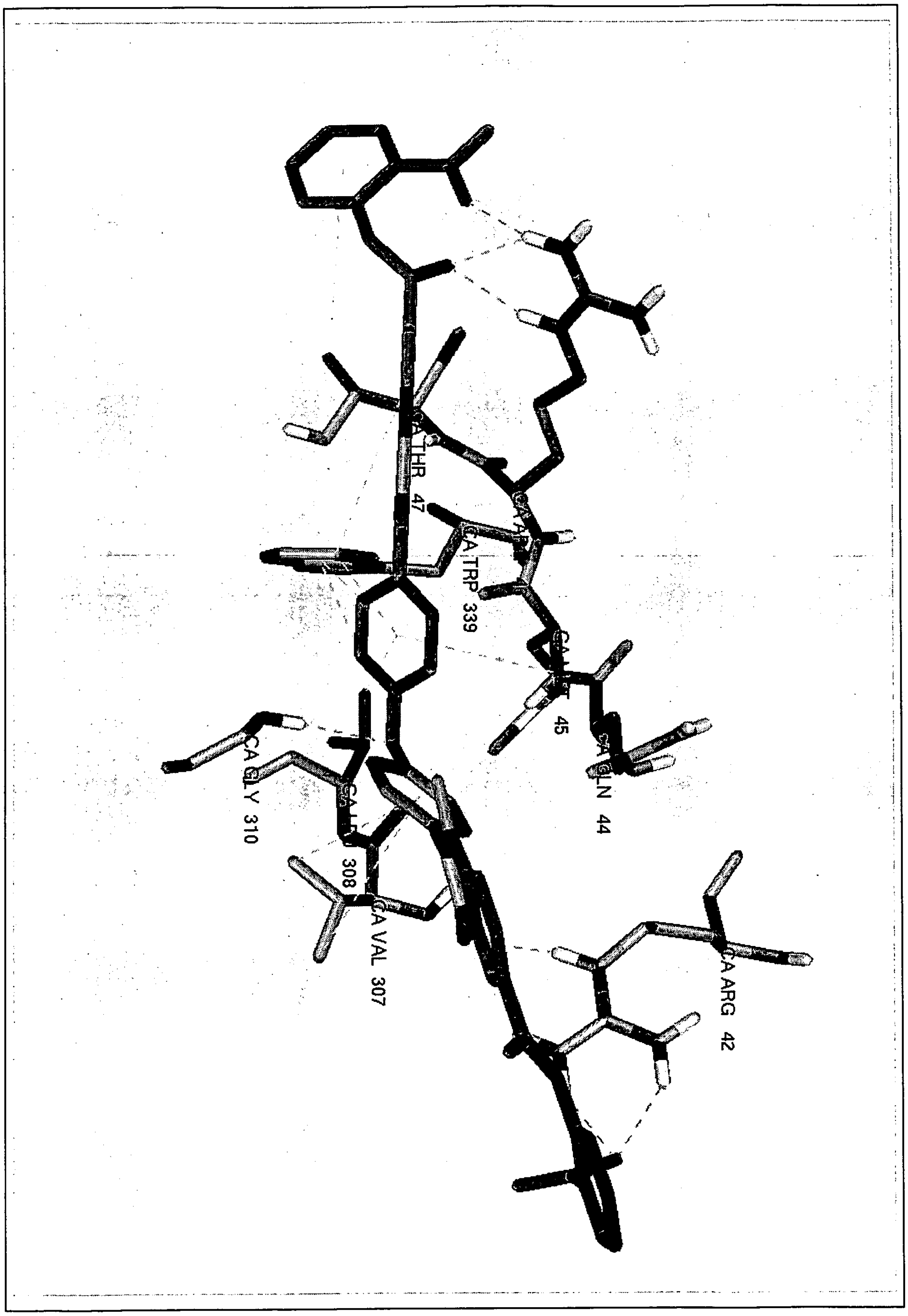


Fig. 11

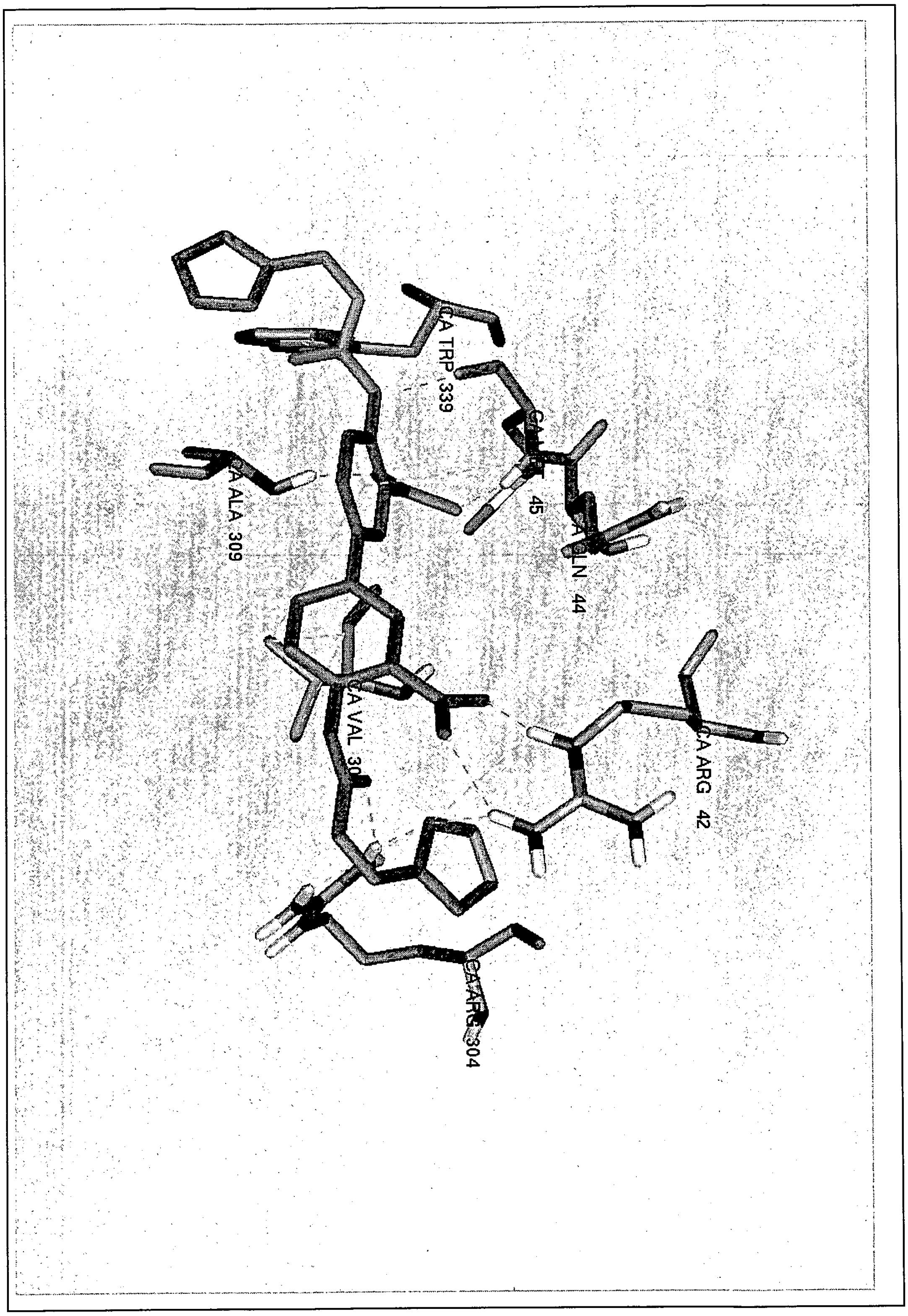


Fig. 12

Figure 13

| | | | | | | | |
|---|---|---|---|---|---|---|---|
| ARG | B | 42 | 177.774 | 57.087 | 55.627 | 1.00 | 14.47 | N |
| ARG | B | 42 | 177.857 | 56.649 | 57.009 | 1.00 | 11.91 | C |
| ARG | B | 42 | 178.109 | 57.909 | 57.813 | 1.00 | 9.77 | C |
| ARG | B | 42 | 179.036 | 58.651 | 57.515 | 1.00 | 7.01 | O |
| ARG | B | 42 | 179.020 | 55.669 | 57.161 | 1.00 | 14.78 | C |
| ARG | B | 42 | 179.169 | 55.055 | 58.534 | 1.00 | 18.79 | C |
| ARG | B | 42 | 180.165 | 53.913 | 58.493 | 1.00 | 24.97 | C |
| ARG | B | 42 | 179.598 | 52.718 | 59.112 | 1.00 | 36.64 | N |
| ARG | B | 42 | 179.080 | 51.678 | 58.456 | 1.00 | 42.55 | C |
| ARG | B | 42 | 179.055 | 51.649 | 57.120 | 1.00 | 42.16 | N |
| ARG | B | 42 | 178.541 | 50.675 | 59.148 | 1.00 | 47.65 | N |
| ILE | B | 43 | 177.248 | 58.176 | 58.784 | 1.00 | 5.38 | N |
| ILE | B | 43 | 177.371 | 59.373 | 59.603 | 1.00 | 8.73 | C |
| ILE | B | 43 | 178.317 | 59.138 | 60.766 | 1.00 | 5.55 | C |
| ILE | B | 43 | 178.022 | 58.329 | 61.634 | 1.00 | 8.13 | O |
| ILE | B | 43 | 175.973 | 59.818 | 60.164 | 1.00 | 9.81 | C |
| ILE | B | 43 | 174.998 | 60.068 | 59.013 | 1.00 | 2.00 | C |
| ILE | B | 43 | 176.105 | 61.059 | 61.075 | 1.00 | 4.42 | C |
| ILE | B | 43 | 175.534 | 60.948 | 57.925 | 1.00 | 2.00 | C |
| GLN | B | 44 | 179.428 | 59.875 | 60.807 | 1.00 | 8.50 | N |
| GLN | B | 44 | 180.407 | 59.739 | 61.884 | 1.00 | 2.00 | C |
| GLN | B | 44 | 180.808 | 61.085 | 62.467 | 1.00 | 2.00 | C |
| GLN | B | 44 | 181.742 | 61.657 | 62.033 | 1.00 | 2.00 | O |
| GLN | B | 44 | 181.648 | 58.996 | 61.393 | 1.00 | 2.00 | C |
| GLN | B | 44 | 181.296 | 57.664 | 60.733 | 1.00 | 16.49 | C |
| GLN | B | 44 | 182.493 | 56.804 | 60.368 | 1.00 | 16.27 | C |
| GLN | B | 44 | 182.658 | 55.705 | 60.906 | 1.00 | 21.55 | O |
| GLN | B | 44 | 183.313 | 57.278 | 59.427 | 1.00 | 19.83 | N |
| MET | B | 45 | 180.177 | 61.500 | 63.558 | 1.00 | 2.57 | N |
| MET | B | 45 | 180.507 | 62.776 | 64.173 | 1.00 | 2.01 | C |
| MET | B | 45 | 181.636 | 62.660 | 65.181 | 1.00 | 3.86 | C |
| MET | B | 45 | 181.777 | 61.662 | 65.887 | 1.00 | 2.82 | O |
| MET | B | 45 | 179.269 | 63.374 | 64.826 | 1.00 | 2.00 | C |
| MET | B | 45 | 178.189 | 63.621 | 63.828 | 1.00 | 2.00 | C |
| MET | B | 45 | 176.669 | 64.119 | 64.557 | 1.00 | 4.65 | S |
| MET | B | 45 | 176.051 | 62.558 | 65.075 | 1.00 | 15.17 | C |
| ARG | B | 46 | 182.398 | 63.733 | 65.289 | 1.00 | 6.01 | N |
| ARG | B | 46 | 183.536 | 63.799 | 66.171 | 1.00 | 2.00 | C |
| ARG | B | 46 | 183.346 | 64.763 | 67.325 | 1.00 | 2.45 | C |
| ARG | B | 46 | 182.722 | 65.815 | 67.175 | 1.00 | 2.95 | O |
| ARG | B | 46 | 184.745 | 64.209 | 65.350 | 1.00 | 2.00 | C |
| ARG | B | 46 | 186.021 | 64.285 | 66.117 | 1.00 | 3.00 | C |
| ARG | B | 46 | 187.195 | 64.254 | 65.189 | 1.00 | 4.11 | C |
| ARG | B | 46 | 188.439 | 64.231 | 65.943 | 1.00 | 11.83 | N |
| ARG | B | 46 | 189.633 | 64.091 | 65.385 | 1.00 | 17.61 | C |
| ARG | B | 46 | 189.722 | 63.961 | 64.064 | 1.00 | 14.81 | N |
| ARG | B | 46 | 190.731 | 64.109 | 66.142 | 1.00 | 17.07 | N |
| THR | B | 47 | 183.901 | 64.412 | 68.474 | 1.00 | 2.00 | N |
| THR | B | 47 | 183.820 | 65.271 | 69.651 | 1.00 | 5.52 | C |
| THR | B | 47 | 184.714 | 66.487 | 69.407 | 1.00 | 4.12 | C |
| THR | B | 47 | 185.863 | 66.327 | 69.034 | 1.00 | 6.06 | O |
| THR | B | 47 | 184.300 | 64.521 | 70.933 | 1.00 | 2.65 | C |
| THR | B | 47 | 183.391 | 63.453 | 71.230 | 1.00 | 2.00 | O |
| THR | B | 47 | 184.375 | 65.477 | 72.139 | 1.00 | 2.51 | C |
| ARG | B | 304 | 169.769 | 52.308 | 59.685 | 1.00 | 14.56 | N |
| ARG | B | 304 | 170.682 | 53.030 | 60.552 | 1.00 | 15.76 | C |
| ARG | B | 304 | 171.733 | 53.641 | 59.651 | 1.00 | 11.57 | C |
| ARG | B | 304 | 172.320 | 52.948 | 58.830 | 1.00 | 9.89 | O |

Figure 13 (cont.)

| | | | | | | | | |
|---|---|---|---|---|---|---|---|---|
| ARG | B | 304 | 171.366 | 52.106 | 61.562 | 1.00 | 20.65 | C |
| ARG | B | 304 | 170.578 | 51.834 | 62.825 | 1.00 | 26.96 | C |
| ARG | B | 304 | 171.496 | 51.865 | 64.049 | 1.00 | 37.55 | C |
| ARG | B | 304 | 172.312 | 50.659 | 64.236 | 1.00 | 42.53 | N |
| ARG | B | 304 | 173.586 | 50.540 | 63.862 | 1.00 | 42.43 | C |
| ARG | B | 304 | 174.203 | 51.552 | 63.267 | 1.00 | 41.50 | N |
| ARG | B | 304 | 174.259 | 49.426 | 64.127 | 1.00 | 41.44 | N |
| ALA | B | 305 | 171.941 | 54.943 | 59.792 | 1.00 | 7.15 | N |
| ALA | B | 305 | 172.939 | 55.666 | 59.013 | 1.00 | 5.46 | C |
| ALA | B | 305 | 174.256 | 55.737 | 59.765 | 1.00 | 4.21 | C |
| ALA | B | 305 | 175.310 | 55.956 | 59.162 | 1.00 | 10.31 | O |
| ALA | B | 305 | 172.451 | 57.080 | 58.700 | 1.00 | 3.09 | C |
| GLY | B | 306 | 174.194 | 55.608 | 61.088 | 1.00 | 8.26 | N |
| GLY | B | 306 | 175.403 | 55.683 | 61.886 | 1.00 | 4.42 | C |
| GLY | B | 306 | 175.150 | 55.502 | 63.367 | 1.00 | 6.47 | C |
| GLY | B | 306 | 174.037 | 55.205 | 63.777 | 1.00 | 10.84 | O |
| VAL | B | 307 | 176.192 | 55.704 | 64.158 | 1.00 | 3.46 | N |
| VAL | B | 307 | 176.157 | 55.543 | 65.604 | 1.00 | 9.87 | C |
| VAL | B | 307 | 176.789 | 56.803 | 66.204 | 1.00 | 10.94 | C |
| VAL | B | 307 | 177.860 | 57.214 | 65.761 | 1.00 | 21.75 | O |
| VAL | B | 307 | 177.014 | 54.307 | 66.012 | 1.00 | 8.02 | C |
| VAL | B | 307 | 177.551 | 54.461 | 67.404 | 1.00 | 17.10 | C |
| VAL | B | 307 | 176.186 | 53.044 | 65.934 | 1.00 | 12.86 | C |
| LEU | B | 308 | 176.135 | 57.434 | 67.176 | 1.00 | 9.57 | N |
| LEU | B | 308 | 176.697 | 58.631 | 67.803 | 1.00 | 7.85 | C |
| LEU | B | 308 | 177.376 | 58.196 | 69.097 | 1.00 | 9.48 | C |
| LEU | B | 308 | 176.780 | 57.476 | 69.904 | 1.00 | 16.22 | O |
| LEU | B | 308 | 175.601 | 59.650 | 68.096 | 1.00 | 10.88 | C |
| LEU | B | 308 | 175.891 | 61.164 | 68.176 | 1.00 | 15.36 | C |
| LEU | B | 308 | 175.001 | 61.804 | 69.259 | 1.00 | 9.06 | C |
| LEU | B | 308 | 177.344 | 61.471 | 68.484 | 1.00 | 12.23 | C |
| ALA | B | 309 | 178.646 | 58.579 | 69.248 | 1.00 | 6.07 | N |
| ALA | B | 309 | 179.475 | 58.269 | 70.416 | 1.00 | 2.78 | C |
| ALA | B | 309 | 179.955 | 56.826 | 70.507 | 1.00 | 9.80 | C |
| ALA | B | 309 | 180.053 | 56.279 | 71.597 | 1.00 | 10.48 | O |
| ALA | B | 309 | 178.785 | 58.657 | 71.694 | 1.00 | 2.00 | C |
| GLY | B | 310 | 180.166 | 56.207 | 69.343 | 1.00 | 10.26 | N |
| GLY | B | 310 | 180.685 | 54.841 | 69.228 | 1.00 | 8.08 | C |
| GLY | B | 310 | 180.182 | 53.705 | 70.110 | 1.00 | 10.79 | C |
| TRP | B | 339 | 179.344 | 67.334 | 69.278 | 1.00 | 2.17 | N |
| TRP | B | 339 | 179.746 | 66.559 | 68.106 | 1.00 | 2.00 | C |
| TRP | B | 339 | 179.413 | 67.233 | 66.763 | 1.00 | 2.00 | C |
| TRP | B | 339 | 178.307 | 67.736 | 66.572 | 1.00 | 4.01 | O |
| TRP | B | 339 | 179.088 | 65.162 | 68.155 | 1.00 | 2.00 | C |
| TRP | B | 339 | 179.148 | 64.466 | 69.505 | 1.00 | 2.85 | C |
| TRP | B | 339 | 178.408 | 64.767 | 70.615 | 1.00 | 2.00 | C |
| TRP | B | 339 | 179.961 | 63.335 | 69.857 | 1.00 | 4.39 | C |
| TRP | B | 339 | 178.708 | 63.901 | 71.633 | 1.00 | 2.00 | N |
| TRP | B | 339 | 179.653 | 63.006 | 71.204 | 1.00 | 3.61 | C |
| TRP | B | 339 | 180.912 | 62.560 | 69.178 | 1.00 | 2.40 | C |
| TRP | B | 339 | 180.265 | 61.935 | 71.879 | 1.00 | 2.10 | C |
| TRP | B | 339 | 181.520 | 61.496 | 69.846 | 1.00 | 2.00 | C |
| TRP | B | 339 | 181.191 | 61.195 | 71.186 | 1.00 | 2.00 | C |

European Patent Office

# EUROPEAN SEARCH REPORT

Application Number
EP 03 01 1263

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int.Cl.7) |
| A | WO 03/022882 A (BAUMGARTNER CHRISTINE ;PROCORDE GMBH (DE); ROSPORT KAI (DE); LOHSE) 20 March 2003 (2003-03-20)<br>* claims 1,10 * | 1 | C07C233/81<br>C07D403/12<br>C07D407/12<br>A61K31/167<br>A61K31/341<br>A61K31/4035<br>G06F17/00<br>G06F19/00 |
| A | EP 0 896 965 A (BOEHRINGER MANNHEIM GMBH) 17 February 1999 (1999-02-17)<br>* column 1, line 1 - column 2, line 2; claims 1,9 * | 1 | |
| A,D | GAUDET R ET AL: "CRYSTAL STRUCTURE AT 2.4 A RESOLUTION OF THE COMPLEX OF TRANSDUCIN BETAGAMMA AND ITS REGULATOR, PHOSDUCIN" CELL, CELL PRESS, CAMBRIDGE, NA, US, vol. 87, 1 November 1996 (1996-11-01), pages 577-588, XP002045814<br>ISSN: 0092-8674<br>* the whole document * | 1 | |
| A | FR 2 758 562 A (ADIR) 24 July 1998 (1998-07-24)<br>* claim 1 * | 1 | TECHNICAL FIELDS SEARCHED (Int.Cl.7) |
| A | WO 00/39077 A (GARG NEERAJ ;LI YI LIN (SE); KAROBIO AB (SE); KOEHLER KONRAD (SE);) 6 July 2000 (2000-07-06)<br>* claim 1 * | 1 | C07C<br>C07D<br>A61K<br>G06F |
| A | WO 00/58277 A (AMERICAN HOME PROD) 5 October 2000 (2000-10-05)<br>* claim 1 * | 1 | |
| A | DE 25 52 609 A (INST FRANCAIS DU PETROL) 26 May 1976 (1976-05-26)<br>* claim 1 * | 1 | |
| | -/-- | | |

~~The present search report has been drawn up for all claims~~

| Place of search | Date of completion of the search | Examiner |
|---|---|---|
| Munich | 6 October 2003 | Wörth, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 03 01 1263

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | US 1 522 196 A (CAMPBELL MACMILLAN) 6 January 1925 (1925-01-06) * claim 1 * ----- | 1 | |
| A | US 2 632 701 A (ALLEN CHARLES F H ET AL) 24 March 1953 (1953-03-24) * claim 1 * ----- | 1 | |
| | | | TECHNICAL FIELDS SEARCHED (Int.Cl.7) |

~~The present search report has been drawn up for all claims~~

| Place of search | Date of completion of the search | Examiner |
|---|---|---|
| Munich | 6 October 2003 | Wörth, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

Application Number
EP 03 01 1263

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-15, 24-29

European Patent Office

**LACK OF UNITY OF INVENTION SHEET B**

Application Number
EP 03 01 1263

The Search Division considers that the present European patent application does not comply with the requirements of unity of invention and relates to several inventions or groups of inventions, namely:

1. claims: 1-15 and 24-29

   subject-matter related to compounds of formula A-L-B for use as medicine

   ---

2. claims: 16-23

   subject-matter related to a computer readable database comprising a representation of compounds of formula A-L-N

   ---

## ANNEX TO THE EUROPEAN SEARCH REPORT ON EUROPEAN PATENT APPLICATION NO. EP 03 01 1263

This annex lists the patent family members relating to the patent documents cited in the above-mentioned European search report. The members are as contained in the European Patent Office EDP file on
The European Patent Office is in no way liable for these particulars which are merely given for the purpose of information.

06-10-2003

| Patent document cited in search report | | Publication date | Patent family member(s) | | | Publication date |
|---|---|---|---|---|---|---|
| WO 03022882 | A | 20-03-2003 | US | 2003049258 | A1 | 13-03-2003 |
| | | | WO | 03022882 | A2 | 20-03-2003 |
| EP 0896965 | A | 17-02-1999 | EP | 0896965 | A1 | 17-02-1999 |
| | | | AU | 7427098 | A | 29-09-1998 |
| | | | WO | 9840402 | A2 | 17-09-1998 |
| FR 2758562 | A | 24-07-1998 | FR | 2758562 | A1 | 24-07-1998 |
| | | | AU | 5993198 | A | 07-08-1998 |
| | | | WO | 9831689 | A1 | 23-07-1998 |
| | | | ZA | 9800441 | A | 29-07-1998 |
| WO 0039077 | A | 06-07-2000 | AU | 758202 | B2 | 20-03-2003 |
| | | | AU | 1885500 | A | 31-07-2000 |
| | | | BR | 9916851 | A | 16-10-2001 |
| | | | CA | 2356319 | A1 | 06-07-2000 |
| | | | CN | 1337953 | T | 27-02-2002 |
| | | | CZ | 20012204 | A3 | 14-11-2001 |
| | | | EP | 1144370 | A2 | 17-10-2001 |
| | | | HU | 0104666 | A2 | 28-03-2002 |
| | | | WO | 0039077 | A2 | 06-07-2000 |
| | | | JP | 2002533432 | T | 08-10-2002 |
| | | | NO | 20012931 | A | 21-08-2001 |
| | | | TR | 200101834 | T2 | 21-12-2001 |
| | | | ZA | 200104932 | A | 15-01-2003 |
| WO 0058277 | A | 05-10-2000 | AU | 4045800 | A | 16-10-2000 |
| | | | WO | 0058277 | A1 | 05-10-2000 |
| | | | US | 2002111369 | A1 | 15-08-2002 |
| | | | US | 6355633 | B1 | 12-03-2002 |
| DE 2552609 | A | 26-05-1976 | FR | 2291999 | A1 | 18-06-1976 |
| | | | BE | 835801 | A1 | 21-05-1976 |
| | | | DE | 2552609 | A1 | 26-05-1976 |
| | | | GB | 1522197 | A | 23-08-1978 |
| | | | GB | 1522196 | A | 23-08-1978 |
| | | | JP | 51076232 | A | 01-07-1976 |
| | | | NL | 7513771 | A | 28-05-1976 |
| US 1522196 | A | 06-01-1925 | NONE | | | |
| US 2632701 | A | 24-03-1953 | NONE | | | |

EPO FORM P0459

For more details about this annex : see Official Journal of the European Patent Office, No. 12/82